# EUROPEAN PATENT SPECIFICATION

(11) **EP 1 539 205 B1**
(45) Date of publication and mention of the grant of the patent: **23.12.2009**
(21) Application number: 03792934.6
(22) Date of filing: 20.08.2003
(51) Int. Cl.: A61K 36/236, A61P 5/24, A61P 15/02, A61P 15/00, A61P 25/28

(54) **PHYTOPROGESTOGENIC EXTRACTS FROM RHIZOMA LIGUSTICUM CHUANXIONG AND USES THEREOF**
PHYTOPROGESTOGENEN EXTRAKTEN AUS RHIZOMA LIGUSTICUM CHUANXIONG UND IHRE VERWENDUNGSZWECKE
EXTRAITS PHYTOPROGESTOGENIQUES DU RHIZOME DE LIGUSTICUM CHUANXIONG ET LEURS UTILISATIONS

(30) Priority: 20.08.2002 US 404397 P
(43) Date of publication of application: 15.06.2005
(73) Proprietor: NATIONAL UNIVERSITY OF SINGAPORE, Singapore 119260 (SG)
(72) Inventor: YONG, Eu, Leong, Singapore 257718 (SG); LIM, Lis Sa, Elissa, Singapore 518671 (SG); BUTLER, Mark, Stuart, Singapore 138971 (SG)
(74) Representative: Howard, Paul Nicholas
(86) International application number: PCT/SG2003/000195
(87) International publication number: WO 2004/017983

(56) References cited:
- US-A- 4 708 949
- DATABASE WPI Week 199747 Derwent Publications Ltd., London, GB; AN 1997-503754 XP002435256 & CN 1 123 165 A (HU H) 29 May 1996 (1996-05-29)
- DATABASE WPI Week 200280, Derwent Publications Ltd., London, GB; Class B04, AN 2002-733730, XP003014695 & CN 1 360 900 A (BAI M) 31 July 2002
- 'Herbasin Chinese herb database - rhizoma chuanxiong' HERBASIN(SHENYANG) CO. LTD., [Online] 01 February 2002, XP003014697 Retrieved from the Internet: <URL:http://www.herbasin.com/database/chuan gxion.htm> [retrieved on 2003-09-17]
- NAWAZ Z. ET AL.: 'The pure antiestrogen ICI 182,780 inhibits progestin-induced transcription' CANCER RESEARCH vol. 59, 15 January 1999, pages 372 - 376, XP001199777
- KAHMANN S. ET AL.: 'Synergistic enhancement of PRb-Mediated RU486 and R5020 agonist activities through cyclic adenosine 3',5'-monophosphate represents a delayed primary response' MOL. ENDOCRINOLOGY vol. 12, no. 2, February 1998, pages 278 - 289, XP001199776
- DATABASE WPI Week 200140, Derwent Publications Ltd., London, GB; Class B04, AN 2001-375351, XP003014696 & CN 1 256 143 A (SOUTHWEST CHINA DESIGN INST. CHEM. ENG. MIN) 14 June 2000
- ANONYMOUS: "TCM healing center for chronic and difficult diseases: Dysmenorhea" INTERNET CITATION, [Online] 8 June 2002 (2002-06-08), pages 1-3, Retrieved from the Internet: URL:http://web.archive.org/web/20020806182 243/http://www.tcmtreatment.com/images/dis ease/dysmenorrhea.htm>
- DATABASE MEDLINE [Online] US NATIONAL LIBRARY OF MEDICINE (NLM), BETHESDA, MD, US 1992 Database accession no. MLM1291208 & CHEN K.J.; CHEN K.: 'Ischemic stroke treated with Ligusticum chuanxiong' CHINESE MEDICAL JOURNAL vol. 105, no. 10, 1992, pages 870 - 873
- GONG X.; SUCHER N.J.: 'Stroke therapy in traditional Chinese medicine (TCM): Prospects for drug discovery and development' TRENDS IN PHARMACOLOGICAL SCIENCES vol. 20, no. 2, 1999, pages 191 - 196
- LI H.X. ET AL: 'Separation and identification of the phthalic anhydride derivatives of Liqusticum Chuanxiong Hort by GC-MS, TLC, HPLC-DAD, and HPLC-MS.' J CHROMATOGR SCI. vol. 40, no. 3, 2002, pages 156 - 161, XP008084564

## Description

### Field of invention

The present disclosure relates to alcoholic extracts of *Rhizoma Ligusticum Chuanxiong* and their uses for conditions requiring progestogen therapy.

### Prior art and background of the invention

Progesterone (PG) is a steroid hormone that has an essential role in mammalian reproduction. Together with estrogen, progesterone acts on the central nervous system, ovary and uterus, to initiate changes in the female reproductive tract that are critical for fertilization of the oocyte, implantation of the embryo and maintenance of pregnancy. Progesterone is formed in the corpus luteum, adrenals, testes, and placenta during pregnancy, and regulates the proliferation, differentiation and function of the uterus, mammary gland and ovary (Clarke and Sutherland 1990). In the uterus the hormone transforms the endometrium from a proliferative to a secretory phase and together with estradiol maintains endometrial integrity in the luteal phase of the menstrual cycle and in pregnancy. Progesterone also inhibits myometrial contractility and has a key role in mammary gland development and lactation. The diverse physiological effects of progesterone are however not restricted to the female reproductive tract. Progesterone has also been implicated in the function of the cardiovascular, immune, bone and central nervous systems. Progestogens act by binding specifically to the progesterone receptor in responsive cells. The receptor-ligand complex then translocates to the cell nucleus, binds to chromosomal DNA in the promoters of progesterone-responsive genes thereby regulating gene transcription. More recently, non-receptor mediated actions of progesterone have also been described.

The progesterone receptor is a member of the steroid/nuclear receptor superfamily of transcription factors (Nuclear Receptor Nomenclature Committee, 1999). The 50 or so proteins of this superfamily are involved in diverse physiological functions such as control of embryonic development, cell differentiation, and homeostasis. The progesterone receptor is encoded by a single copy gene on chromosome 11q 22-23 and has with 8 exons spanning more than 90 Kb (Misrahi *et al* 1993, Rousseeu-Merck *et al* 1987). There are 2 isoforms of the progesterone receptor, PR-A (85 KDa) and PR-B (115 KDa). PR-A and PR-B differ, in that PR-B has an additional stretch of 164 amino acids located at the amino terminus of the receptor. When both receptors are expressed in equimolar quantities, PR-A and PR-B can heterodimerize and transactivate. Like other steroid receptors, the progesterone receptor has four main functional domains, namely the N-terminal Transactivation domain, followed by the Hinge, DNA-binding, and Ligand-binding domains. The most conserved domain is the DNA-binding domain. On activation by ligand, the DNA-binding domain binds to progesterone-responsive elements (PRE) which are composed of two palindromic sequences. The consensus PRE sequence is TGTACAnnnTGTTCT, where n represents any nucleotide. The carboxyl terminal domain is the ligand-binding domain (Bourguet *et al* 1995, Carson-Jurica *et al* 1990) which in the presence of progesterone causes nuclear translocation, dimerization and formation of the pre-initiation complex on PRE of progesterone-responsive genes.

The function of progesterone acting through its receptor system is indicated by anovulatory diseases in women where the hormone is lacking. The use of mouse mutants in which expression of individual progesterone receptor genes has been specifically ablated has also led to increased appreciation of the diverse roles of progesterone. Female mice lacking both PR gene isoforms exhibit impaired sexual behaviour, impaired neuroendocrine gonadotrophin regulation, anovulation, uterine dysfunction, and impaired ductal branching and lobuloalveolar differentiation of the mammary gland. Progesterone receptors also play an essential role in regulating thymic involution during pregnancy and in the cardiovascular system through regulation of endothelial cell proliferation. Receptors for progesterone have also been identified in the central nervous system and bone suggesting that the hormone is involved in regulating cognitive function and bone maintenance.

### Uterine development and function

Progesterone hormone exerts both proliferative and differentiative effects on the uterus. Progesterone receptors are expressed in epithelial, stromal and myometrial compartments of the uterus (Tibbets *et al* 1998). In non-pregnant women, progesterone promotes changes in the estrogen-primed endometrium in order to convert the endometrium into a secretory phase. Withdrawal of progesterone typically induces endometrial sloughing. The lack of progesterone in anovulatory conditions like perimenopause or polycystic ovarian syndrome can result in hyperplasia and cancer of the endometrium due to the action of unopposed estrogen.

### Mammary Gland

Progesterone and estrogens are the primary steroids required in normal breast development. In pregnancy, progesterone controls the increase in the branching, alveolar proliferation as well as differentiation of alveolar lobules. Studies with the PRKO mouse model have confirmed this action of progesterone (Lydon *et al* 1995).

### Immunity

There are also reports in the prior art that estradiol has a strong pro-inflammatory action in the uterus, while progesterone has a potent anti-inflammatory response. It has been shown that PG can down-regulate the expression of Interleukin 8 (IL-8). The anti-inflammatory role of PR both of which are anti-estradiol and anti-inflammatory is likely to be essential for generation of an immunologically privileged tissue to facilitate implantation of the developing embryo and prevention of embryonic rejection (Ito *et al* 1994, Tibbetts *et al* 1999).

### CNS effects

Estrogen and progesterone, long considered for their roles as primary hormones in reproductive and maternal behavior, are now also being studied as neuroprotective and neuroregenerative agents in stroke and traumatic brain injuries (Stein, 2001). The hormones affect neuronal function including neurobehavioural expression associated with sexual responsiveness (Lydon *et al* 1995) and can prevent of the death of motor neurons (Yu 1988a). Collectively, the hormones reduce the consequences of the injury cascade by enhancing antioxidant mechanisms, reducing excitotoxicity (altering glutamate receptor activity, reducing immune inflammation, providing neurotrophic support, stimulating axonal remyelinization), and enhancing synaptogenesis and dendritic arborization. Whereas estrogen seems more effective as a prophylactic treatment in females at risk for cardiac and ischemic brain injury, progesterone appears to be more helpful in the post-injury treatment of both male and female subjects with acute traumatic brain damage.

Compounds with biological activity similar to progesterone are known as progestogens, progestins or gestogens. The majority of progestogens are steroidal compounds belonging to progesterone and its derivatives, and testosterone and 19-nortestosterones and their derivatives. A smaller number of progestogens that are non-steroidal are also known including ligands based on sesquiterpenes possessing a ligularenolid skeleton (Kurihara, 1999), tetrahydropyridazines RWJ 26819 (Palmer, 2000), 5-Aryl-1,2,3,4-tetrahydrochromeno[3,4-]quinolin-3-ones (Zhi *et al* 1999) and the fluorinated phthalides (Lehman *et al* 2001).

Progestins have a wide range of applications such as in oral contraception, hormone replacement therapy (in combination with estrogen) and to assist reproductive technology (Sengupta and Ghosh 2000). Progestins are also used to treat inoperable endometrial cancer and have been suggested to aid in the reduction of risk to endometrial cancer from postmenopausal estrogen in woman. Effective treatment of secondary amenorrhea, functional uterine bleeding as well as related menstrual disorders (caused by hormonal deficiency or imbalance) have been achieve with the use of progestins.

Progestogens are commonly used in hormone replacement therapy, normally in conjunction with estrogen to protect the endometrium from hyperplasia and cancer (Skouby 2001). High doses of progestogens are used to treat irregular and abnormal menstrual bleeding, to suppress endometrial growth, and for treating endometrial hyperplasia. Progestogens have anti-neoplastic activity against endometrial hyperplasia and cancer. Other uses for progestogens include treatment of premenstrual symptoms such as headaches, depression, water retention and mastodynia. Progestogens alone or in combination with other steroid hormones are also essential for the treatment of endometriosis and are essential components in the composition of female birth control pills and hormone replacement therapy. Progestogens are also used to treat luteal phase defects and for luteal, support in assisted reproduction cycles for infertile patients. After conception, progestogens are used for fetal support.

Presently, the progestogens available commercially comprise synthetic products such as medroxyprogesterone acetate and progesterol acetate. Some of the progestogens available commercially also result in undesirable side effects such as excessive androgen activity. It is therefore important to develop new progestogens which overcome the above drawbacks. In addition, considering the growing popularity for drugs based on traditional systems of medicine across the world, herbs and herbal extracts are of prime importance. Thus, phytoprogestogenic compounds would open new avenues of treatment for many people. Currently there are no known phytoprogestogens and the discovery of such substances would be of great commercial value.

Traditional systems of medicine such as those practiced in China, Korean, Indian, Japanese and other Asian countries offer a wide variety of solutions to several diseases/disorders. Most traditional systems of medicine rely on herbs and herbal extracts and combinations thereof. Some traditional systems of medicine also rely on the use of specific dosages of minerals either alone or in combination with herbs/herbal extracts to provide relief/cure for several disorders.

*Ligusticum* belongs to the *Umbelliferae* family, members of which include *L Chuanxiong* (Chuan Xiong), *L. wallichii, L. sinense* (Gao Ben), and *L. brachylobum.* The dried rhizome of *Ligusticum Chuanxiong* is a very common crude drug in traditional Chinese, Japanese and Korean medicines. In Japan, *Senkyu* is obtained from the dried rhizome of *Ligusticum officinale* KITAGAWA, a variety of *L. wallichii.*

*Ligusticum's* traditional actions include invigorating blood circulation, promoting the flow of Qi, dispelling wind, and alleviating pain. It *Ligusticum* has traditionally been prescribed for headaches, abdominal pain, arthralgias, and menstrual disorders caused due to blood stasis. *L. Chuanxiong* is indicated for menstrual disorders, amenorrhoea, dysmenorrhoea, abdominal pain with mass formation, pricking pain in the chest and costal regions pain due to traumatic injury, headache and rheumatic arthralgia (Chinese Pharmacopoeia, 1997).

Over 90 essential oils (Zhong *et al* 1996) and 20 phthalides (Naito *et al* 1992) have been isolated from *Ligusticum* species and their value to the plant is believed to be because some of these alkylphthalide derivatives have antimicrobial, anti-fungal, anti-helminthic activities (Sinclair, 1998). Major constituents include ligustilide, butylidenephthalide, cnidilide, neocnidilide and butylphthalide. Pregnenolone, coniferylferulate, senkyunolide-A and their mono- and dihydroxyphthalide derivatives have also been found. Other ingredients include an alkaloid, tetramethylpyrazine, ferulic acid (a phenolic compound), chrysophanol, sedanoic acid, and 1-2 % of essential oils such as ligustilide and butylphthalide. Significant amounts of minor components can be derived from the original components subsequent to processing and storage. For example the dihydroxyphthalides senkyunolide-H and I and senkyunolide-J can be synthesized from major components ligustilide and senkyunolide-A. These oxygenated phthalides were absent from the fresh herb but were shown to be derived from the major volatile phthalides during storage of the crude drug. Similarly ferulic acid is a decomposition product from coniferylferulate (Kobayashi *et al* 1984).

### Animal studies

### Car-diovascular effects

Four phthalide dimers from *Ligusticum Chuanxiong,* tokinolide B, levistolide A, ligustilide and senkyunolide P are shown to relax KCl-induced contraction on rat thoracic aorta and reduced KCl-induced perfusion pressure of rat mesenteric arteries. Coniferylferulate reduced methoxamine-induced perfusion pressure on rat mesenteric arteries. On the other hand the phthalide dimers, tokinolode B and senkyunolide P and the phthalides, senkyunolide, buthylphtalide and cnidilide decrease blood viscosity. These suggest that the herb has important functions for activating the blood circulation and removing blood stasis (Naito *et al* 1995).

Butylidenephthalide was reported to have a selective anti-anginal effect without changing blood pressure. Experiments performed to determine the mechanism of this action suggest that Butylidenephthalide inhibits calcium release from calcium stores more selectively than calcium influx from extracellular space via voltage-dependent calcium channels. The inhibition by butylidenephthalide of calcium release from KCl-sensitive calcium stores might be similar to its inhibition of calcium release from phenylephrine-sensitive calcium stores (Ko *et al* 1998). Senkyu phthalides has also been described as having antiarteriosclerotic properties (Masayasu K *et al* 1989). Thus synthetic butylidenephthalides have been described to have inhibitory effects on mouse aorta smooth muscle cells in-vitro (Mimura *et al* 1995).

The methanol extract from Cnidium rhizome (Senkyu) decreased the contraction and slightly increased the heart rates of the isolated atria. The methanol extract from Cnidium rhizome when fractionated with chloroform and water fractions was reported to exert potent negative inotropic and chronotropic effects in isolated atria. The contraction was attenuated by two major components in the chloroform fraction, ligustilide and senkyunolide, but the heart rates were scarcely affected by these components. The chloroform fraction induced changes in resting potentials and configurations of normal action potentials recorded in the isolated left atria: the resting potentials were depolarized, and the upstroke velocity of the action potentials decreased. Neither ligustilide nor senkyunolide exerted such effects. The upstroke velocity of action potentials recorded in partially depolarized atria was reduced by the chloroform fraction as well as ligustilide and senkyunolide (Nakazawa *et al* 1989).

By occluding the bilateral carotid arteries of rabbits to produce bilateral partial cerebral ischemia, and by using radioimmunoassays to measure the levels of dynorphin A1-13 like immunoreactivity (ir-Dyn A1-13) in plasma and cerebrospinal fluid (CSF), Liu & Shi (1990) found that the levels of ir-Dyn A1-13 in plasma and CSF have significantly increased (P less than 0.01) after cerebral ischemia appears. The result of the *Ligusticum wallichii* Franch (*Ligusticum*) pretreatment to the test-group shows a definite improvement of the changes of ir-Dyn A1-13 levels in plasma and CSF. The severity of brain ischemic damage and neurologic dysfunction in *Ligusticum*-treated animals is lighter than that of saline-treated group, too. In this study, some new approaches are explored to explain the pathophysiology of cerebral ischemia and the mechanisms by which *Ligusticum* prevents and treats cerebral ischemia (Liu & Shi 1990).

### Anti-inflammatory Properties:

In a Japanese study, the active ingredients in *Ligusticum,* tetramethylpyrazine and ferulic acid, were found to have both significant anti-inflammatory and analgesic effects (Ozaki 1992). When given to guinea pigs with histamine/acetylcholine induced bronchospasm, *Ligusticum* was found to decrease plasma levels of thromboxane B2, relax tracheal muscle, increase the forced expiratory volume, and inhibit the synthesis and release of thromboxane A2 with no adverse side-effects. The total effective rate was 92 percent vs. 62 percent in the control group (P <0.01).

### Asthma and Bronchial smooth muscle

*Ligusticum wallichii* mixture was also reported to inhibit bronchospasm induced by histamine and acetylcholine in guinea pigs; to decrease the plasma level of TXB2. The incubation period from antigen inhalation to asthma attack could be delayed by *Ligusticum wallichii* mixture and the incidence of asthma and its mortality were reduced in guinea pigs compared with control, P < 0.01. (Shao *et al* 1994)

### CNS and Anticonvulsive activities

Early reports suggest that 3-n-butyl phthalide can facilitate the performance of learning and memory in rats (Yu *et al* 1988a) and that it has a protective effect on rat brain cells (Yu *et al* 1988b).

### Antibacterial/Antifungal Effects:

*Ligusticum* has demonstrated in vitro effects against several strains of pathogenic bacteria including *Pseudomonas aeruginosa, Shigella sonnei, Salmonella typhi,* and *Vibrio cholera* (Bensky & Gamble 1993). The essential oil components of *Ligusticum* (butylphthalide) have been shown to inhibit dermatophytes in vitro (Hong 1986).

### Renal effects

It is believed that *Ligusticum wallichii* can also affect the toxicity of Cyclosporine A on renal function, renin-angiotensin system and platelet aggregation in Sprague Dolly rats (Liu 1992). Infusion of Cyclosporine A (50 mg/kg, iv) resulted in a significant fall in glomerular filtration rate and renal plasma flow and a significant increase of plasma renin activity, angiotensin II level and percentage platelet aggregation. At the same time, treatment with 20% *Ligusticum wallichii* (8 ml/kg, iv) before cyclosporine A infusion significantly prevented the decline of glomerular filtration rate and renal plasma flow as well as the enhancement of platelet aggregation, but had no influence on cyclosporine A mediated renin-angiotensin system activation. These results suggested that *Ligusticum wallichii* may be beneficial to the acute nephrotoxicity induced by cyclosporine A (Liu 1992).

### Skin permeability

*Ligustici Chuanxiong Rhizoma* (Senkyu) ether extract has been reported to enhanced permeability of moderately lipophilic compounds into the skin. (Sekiya *et al* 1997*,* Nmaba *et al* 1992).

### Human studies on Ligusticum herbs

### Cerebro-vascular and coagulation effects

*Ligusticum* has been studied in the treatment of ischemic stroke (Chen 1992b). Some injections of the medicines, including *Ligusticum,* Ligustrazine, Ligustylid and ferulic acid, were tested clinically and experimentally. The results showed that the effects of the drugs were the same as or even better than those of the controls, such as papaverine, dextran and aspirin-persantin. They could improve brain microcirculation through inhibiting thrombus formation and platelet aggregation as well as blood viscosity.

One hundred and fifty-eight subjects with transient ischemic attack were randomly divided into a *Ligusticum* group (111 cases) and an aspirin group (47 cases). The total effective rate in the *Ligusticum* group was 89.2 percent as compared to 61.7 percent in the aspirin group (P<0.01 ) (Chen 1992b). *Ligusticum* increased cerebral blood flow, accelerated the velocity of blood flow, dilated the spastic artery, and decreased peripheral arterial resistance. In another study, *Ligusticum* was evaluated in the treatment of ischemic stroke. Injectable preparations were shown to improve brain microcirculation through inhibiting thrombus formation, decreasing platelet aggregation, and improving blood viscosity. The effect of *Ligusticum* was the same or better than the controls of papaverine, dextran and aspirin-persantin (Chen & Chen 1992).

In another double-blind trial 220 patients with acute cerebral infarction were randomly divided into *Ligusticum Chuanxiong* group (134 cases) and low molecular weight dextran group (86 cases) to evaluate the herb's effects on neurologic function and living capability. The results showed that the total therapeutic efficacy rate in Chuanxiong group and in dextran 40 group were 86.6% and 62.8% respectively. The effect of Chuanxiong on treatment of acute cerebral infarction was superior to low molecular weight dextran. The difference between the two groups was also statistically significant (P <0,01) (Chen 1992a).

By using ELISA and RIA to measure the levels of Beta-thromboglobulin (beta-TG), platelet factor 4, thromboxane B2 and 6-keto-prostaglandin F1 alpha in plasma of patients with acute cerebral infarction, Liu (1991) found that the levels of beta-TG, platelet factor 4 and thromboxane B2 in plasma had significantly increased (P less than 0.01), but the level of 6-keto- prostaglandin F1 alpha in plasma showed no change (P greater than 0.05). The results of the *Ligusticum wallichii* treatment to the test-group showed that the levels of Beta-thromboglobulin, platelet factor 4 and T thromboxane B2 in plasma had significantly decreased (P less than 0.01), and the level of 6-keto- prostaglandin F1 alpha in plasma had significantly increased (P less than 0.05). This suggested that the *Ligusticum* treatment could effectively inhibit the platelet activation in vivo and correct the thromboxane A2-prostaglandin F2 imbalance in blood of the patients (Liu 1991).

### Toxicity:

*Ligusticum* is prescribed in traditional Chinese decoctions at dosages up to 9 grams administered over several days. Overdose symptoms may include vomiting and dizziness (Bensky & Gamble 1993).

US Patent 4,708,949 discloses therapeutic compositions are composed of four plant extracts: ginsenoside, tetramethyl pyrazine, astragalan and atractylol. Pharmaceutical dosage units are prepared by conventional means with specific weight ranges and proportions of each of the four ingredients. The pharmaceutical dosage units are claimed to be highly effective in treating cerebral vascular disease and the sequelae thereof. The dosage units are also useful for bolstering immunofunction in healthy and diseased patients. Tetramethyl pyrazine is preferably extracted from *Ligusticum Chuanxiong.*

There has however been no teaching in the art that associates ***L.C.*** or the *Ligusticum* family or their extracts or compounds contained therein with progestogenic activity.

Many women consider prescription progestogens to be unnatural. As a result, there would be a demand for phyto-progestogens derived from herbs. There is a need to identify naturally occurring compounds which can activate the progesterone receptor and thus can act as phyto-progestogens. Such herbal derived progestogens are needed to develop therapeutic compositions, botanical drugs and/or nutraceutical applications. Pure chemical phyto-progestogens can be developed as pharmaceutical applications.

### REPORTER GENE BIOASSAYS FOR STEROIDOGENIC ACTIVITY

The measurement of steroid bioactivity and bioavailability in animal models is an essential part of preclinical drug evaluation. To determine whether a herbal extract or any other complex mixture is clinically effective, data on its absorption, metabolism and bioavailability in animal models is required. Unlike pure compounds, herbal extracts are mixtures of multiple compounds, many of which contribute to biological activity. Current methods used in the pharmaceutical industry to perform pharmacokinetic/dynamic studies in man or animal models, depend on the measurement and tracking of single compounds in serum using chromatography and mass spectrometric techniques. These conventional techniques are inadequate for studies of botanical or animal-derived drugs since these comprise multiple biologically active compounds. Although there are preliminary reports of bioassays to track androgenic (Paris, 2002), estrogenic (Paris, 2002) and glucocorticoid (Vermeer, 2003) bioactivities in serum using reporter genes in cells, there are no reports of bioassays to measure progestogenic activity. Furthermore, there is a need to develop methods to extract steroid-active small compounds from serum and compared their bioactivity, bioavailability and bioequivalence to reference pharmaceutical compounds in animal models.

### Objects of the invention

The main object of the present invention is to provide herbal extracts with progestogenic activity which substantially replicate the activity of progesterone.

Also disclosed are methods for the screening of herbal extracts to determine their potential progestogenic activity, validate such activity and prepare herbal extracts which can be used as progestogens.

Also disclosed are methods for the preparation of herbal extracts of *Ligusticum Chuanxiong* which can be used as a progestogen in crude extract form or after further purification.

Also disclosed are methods for the preparation of herbal extracts of *Ligusticum Chuanxiong* which after purification provide enriched fractions of extract useful as progestogens.

Also disclosed are uses of extracts containing phyto-progestogens from *Rhizoma Chuanxiong,* to treat health problems requiring progesterone therapy, supplementation or replacement including fetal support, menstrual disorders, amenorrhoea, menorrhagia, polycystic ovarian syndrome, pregnancy complications, endometriosis, contraception, menopause, endometrial hyperplasia and endometrial cancer.

Also disclosed are uses of extracts containing phyto-progestogens from *Rhizoma Chuanxiong,* alone, or in combinations with other compounds, for oral contraception, for hormonal replacement therapy, for treating endometriosis, neuroprotective and neuroregenerative agents in stroke and traumatic brain injuries.

Also disclosed are methods to separate fractions of *Rhizoma Chuanxiong,* enriched for 3-butyl-4,5-dihydrophthalide, 3-butyl-phthalide and components and co-eluates, with strong progestogenic activity using solvent-solvent partitioning, solid-phase fractionations and high-performance liquid chromatography (HPLC).

Also disclosed are methods for standardization and fingerprinting of *Rhizoma Chuanxiong* extracts using solvent-solvent partitioning solid phase fractionations, HPLC, mass spectrometry (MS), and nuclear magnetic resonance imaging (NMR).

Also disclosed are methods for measurement and standardization of the bioactivity *Rhizoma Chuanxiong* and its extracts based on the presence of 3-butyl-4,5-dihydrophthalide and 3-butyl-phthalide.

### Summary of the invention.

The present invention is as defined in the claims.

The present invention is derived from the analysis of various herbs noted in traditional Chinese medicine for their "uterotonic" activity in order to firstly determine which of them may possess progestogenic activity, and secondly to isolate/prepare extracts therefrom with progestogenic activity. The present invention is derived from screening of herbs reported to posses "uterotonic activity" in ancient pharmacopoeia, for progestogenic activity and to purify and characterize pure phyto-progestogens form bioactive herbal extracts.

According we disclose a method for the preparation of an alcoholic extract from *Rhizoma Chuanxiong* useful as a progestogen, said method comprising subjecting powdered *Rhizoma Chuanxiong* to a first extraction with an alcoholic solvent selected from the group consisting of ethanol and methanol, separating a first *Rhizoma Chuanxiong* extract obtained thereby from a supernatant, subjecting the filtered first extract to a second extraction with an alcoholic solvent to obtain a second extract, separating and drying the second extract

In one embodiment of the method, the solvent used for the first extraction is selected from ethanol in a concentration of 70% and 100%.

In another embodiment of the method, the solvent for the first extraction is selected from methanol in a concentration of 70% and 100%.

In another embodiment of the method, in the first extraction, the powdered *Rhizoma Chuanxiong* is mixed with the solvent and then allowed to soak for 5-7 days at 37°C.

In another embodiment of the method, the first extract is separated from the supernatant by filtration using Whatman Grade I (11 µm pore size) filter paper.

In another embodiment of the method, the second extraction is carried out over a time period in the range of 2-3 hours.

In another embodiment of the method, the second extract is separated from the solvent by filtration.

In another embodiment of the method, the separated second extract is dried in a rotary evaporator.

In another embodiment of the method, the solvent used for the second extraction is the same as the solvent used for the first extraction.

In another embodiment of the method, the solvent used for second extraction is selected from the group consisting of 50%, 70% and 100% ethanol in water, and 70% and 100% methanol in water.

In another embodiment of the method, the dried second extract is suspended in an alcoholic medium.

In another embodiment of the method, the medium used for suspending the dried second extract is selected from ethanol and methanol in a concentration of 100%.

In another embodiment of the method, the dried extract is suspended in the alcoholic medium at a concentration in the range of 6.25µg/ml to 100 µg/ml of medium.

In another embodiment of the method, the dried extract is suspended in the alcoholic medium at a concentration of 50 µg/ml.

In another embodiment of the method, the dried extract of *Rhizoma Chuanxiong* contains 3-butyl-4,5-dihydrophthalide and 3-butyl-phthalide as active constituents.

In another embodiment of the method, the 3-butyl-4,5-dihydrophthalide is present in the extract in an amount of about 95% and the 3-butyl-phthalide is present in an amount of about 2%.

In another embodiment of the method, the dried second extract of *Rhizoma Chuanxiong* is subjected to purification to enrich the second extract.

In another embodiment of the method, the purification is carried out by eluting the dried second extract with a solvent using reverse phase extraction in a C18 matrix contained in a glass column.

In another embodiment of the method, the solvent used for elution is selected from the group consisting of 30% methanol, 80% methanol, 100% methanol and DCM.

In another embodiment of the method, the purification is carried out using reverse phase extraction in a Diol matrix contained a glass column.

In another embodiment of the method, the reverse phase extraction is carried out using an eluate selected from the group consisting of 30%EtoAc:70%DCM, 100%DCM and 60%EtoAc:40%DCM.

In another embodiment of the method, the dried extract is first subjected to solvent/solvent partitioning to remove tannins present in said extract.

In another embodiment of the method, solvent/solvent partitioning is carried out by first mixing the dried second extract in ethanol, mixing the ethanolic extract obtained thereby with water to obtain an ethanolic solution, adding hexane to the solution to obtain a first hexane layer, separating the hexane layer and adjusting the ethanol:water ratio in the remaining solution to 3:2, adding equivolume of DCM to obtain a DCM layer, separating the DCM layer, adding butanol to the remaining solution after DCM layer removal to obtain a butanol layer, separating the butanol layer to leave a water layer as remnant, the tannins being partitioned to the butanol layer and the water layer, the prior separated hexane and DCM layers being enriched in *Rhizoma Chuanxiong* extract.

The present invention also relates to the therapeutic use or a therapeutic method for progesterone replacement or supplementation comprising administering to a patient suffering from conditions requiring progesterone replacement or supplementation, a therapeutically effective dose of an extract of *Rhizoma Chuanxiong.*

In one embodiment of the use, the therapeutically effective dose of *Rhizoma Chuanxiong* extract is in the range of 6.25µ/ml to 100µg/ml.

In another embodiment of the use, the therapeutically effective dose of *Rhizoma Chuanxiong* extract is 50µg/ml of crude extract

In another embodiment of the use, the conditions requiring replacement/supplementation of progesterone are selected from the group consisting of menstrual disorders, amenorrhoea, menorrhagia, polycystic ovarian syndrome, pregnancy complications, endometriosis, contraception, menopause, endometrial hyperplasia and hormonal replacement.

In another embodiment of the use, the *Rhizoma Chuanxiong* extract is used alone or in combination with co-elutes from *Rhizoma Chuanxiong.*

The present disclosure also relates to the use of *Rhizoma Ligusticum chuanxiong* extract for or to a method for the treatment of stroke or brain injuries in a subject suffering the same comprising administering to the subject a pharmaceutically effective amount of *Rhizoma Chuanxiong* extract.

In one embodiment of the use, the pharmaceutically effective dose of *Rhizoma Chuanxiong* extract is in the range of 6.25µg/ml to 100µg/ml.

In another embodiment of the use, the as claimed in claim 29 wherein the pharmaceutically effective dose of *Rhizoma Chuanxiong* extract is 50µg/ml of crude extract.

In another embodiment of the use, the *Rhizoma Chuanxiong* extract is used alone or in combination with co-elutes from *Rhizoma Chuanxiong.*

The present disclosure also provides a novel progestogen receptor assay comprising HeLa cells transiently co-transfected with a first plasmid and a second using lipofectamine.

In one embodiment of the assay, the first plasmid comprises of DNA encoding the full length human progesterone receptor (PR-B) and the second plasmid comprises a progesterone reporter gene (PRE2-TATA-LUC) comprising a luciferase reporter gene driven by two copies of the progesterone response element from the aminotransferase gene.

The present disclosure also relates to a method for preparing a kit for conducting an assay for progesterone receptor activity comprising growing HeLa cells in 24-well microtiter plates, infecting the grown HeLa cells with a first plasmid and a second plasmid using lipofectamine, the first plasmid comprising of DNA encoding the full length human progesterone receptor (PR-B), and the second plasmid comprising a progesterone reporter gene (PRE2-TATA-LUC) comprising a luciferase reporter gene driven by two copies of the progesterone response element from the aminotransferase gene, exposing the transfected cells to ligands in RPMI 1640 medium, supplemented with 10% charcoal-stripped fetal calf serum, . 2mM L-glutamine, 0.1mM non-essential amino acids and 1mM sodium pyruvate for 42-48 hours at 37°C in a 5% carbon dioxide incubator, exposing the replicate wells to ethanol vehicle, rinsing the cells with a buffer selected from a PBS buffer and lysis buffer, collecting the cell lysates for measurement of luciferase activity.

The present disclosure also provides a method for conducting assay of progestogenic activity of an extract *Ligusticum chuanxiong* comprising exposing an assay kit comprising transfected cells of He-La transiently co-transfected with a first plasmid and a second using lipofectamine, to increasing doses of progesterone and the extract.

### BRIEF DESCRIPTION OF THE FIGURES

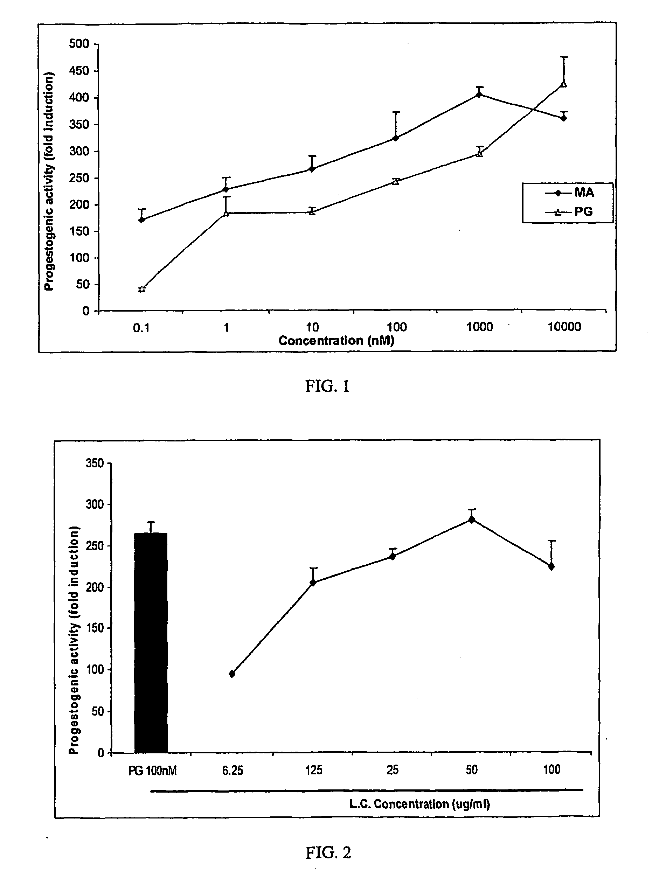

Figure 1 is a graphical representation of dose-response curves of progesterone (PG), and the synthetic progestogen, megesterol acetate (MA); using the cell-based progesterone reporter gene system. Progestogenic activity of the ligands was expressed as fold-increase in luciferase activity compared with control cells exposed to vehicle only. All data points were in triplicate. Error bars represent SEM.
Figure 2 is the dose-response curve of the 100% ethanol extract of *Ligusticum chuanxiong (**L.C.**)* in comparison to PG 100nM. ***L.C.*** extract was prepared as described in example 2, and replicate wells were exposed to increasing concentration of ***L.C.*** extract for 48 hours. Progestogenic activity of the ligands was expressed as fold-increase in luciferase activity compared with control cells exposed to vehicle only. All data points were in triplicate. Error bars represent SEM.

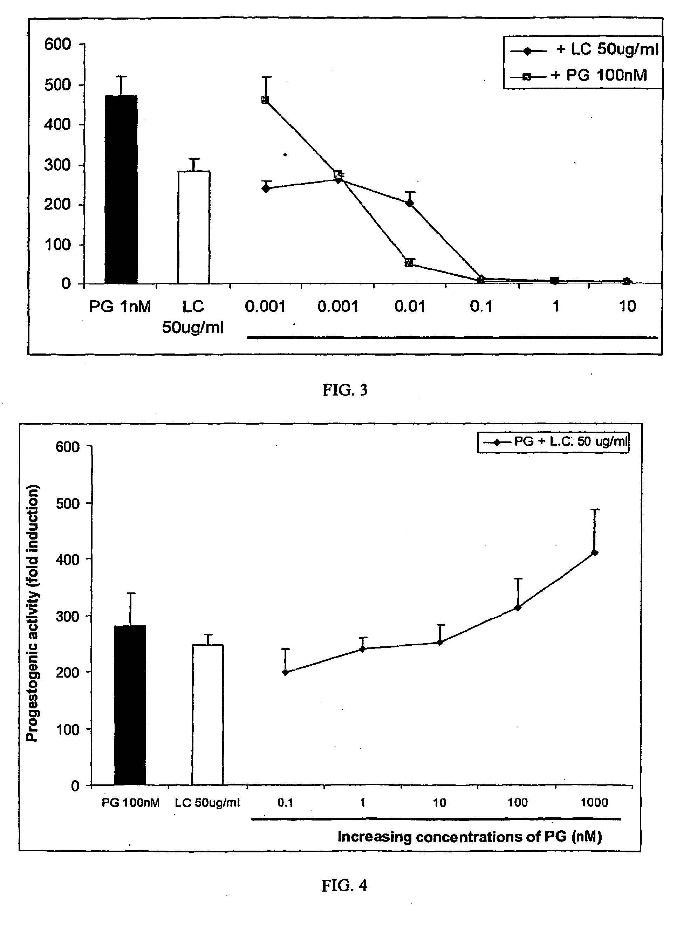

Figure 3 is a representation of the progestogenic activity of ***L.C.*** extract in the presence of a progestogenic antagonist, RU486. Cells were exposed to the indicated concentrations of RU486 in the presence of fixed amounts *of **L.C.*** extract (50µg/ml), or PG 100nM for 48 hours. *L.C.* extract was prepared as described in example 2. Replicate wells exposed to *L.C.* extract (50µg/ml) alone, or PG 100nM alone, served as positive controls. Progestogenic activity of the ligands was expressed as fold-increase in luciferase activity compared with control cells exposed to vehicle only. All data points were in triplicate. Error bars represent SEM.
Figure 4 shows the effect of *L.*C. extract on PG action. Cells were exposed to the indicated concentrations of progesterone (PG) in the presence of fixed amounts of ***L.C.*** extract (50µg/ml). ***L.C.*** extract was prepared as described in example 2. Replicate wells exposed to ***L.C.*** extract (50µg/ml) alone, or PG 100nM alone, served as positive controls. Progestogenic activity of the ligands was expressed as fold-increase in luciferase activity compared with control cells exposed to vehicle only. All data points were in triplicate. Error bars represent SEM.

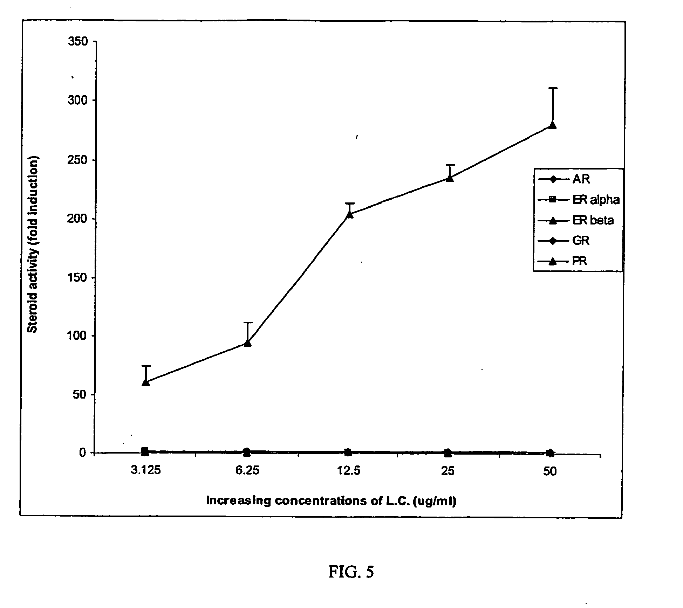

Figure 5 shows the effect *of L.C* extract on glucocorticoid (GR), androgen (AR), estrogen (ERα and ERβ) receptors. Plasmids encoding either PR, GR or AR were coexpressed with a reporter gene (PRE2-TATA-LUC) that contains the hormone-response elements common to all three receptors. Estrogenic activity was measured by expressing ERα or ERβ in the presence of the estrogen-responsive ERE-MMTV-LUC reporter gene. Hormone activity of the ligands was expressed as fold-increase in luciferase activity compared with control cells exposed to vehicle only. All data points were in triplicate. Error bars represent SEM. ***L.C.*** displayed strong progestogenic activity and the absence of activity with the GR, AR, ERα and the ERβ. This indicated that ***L.C.*** has specific agonistic activity for PR.

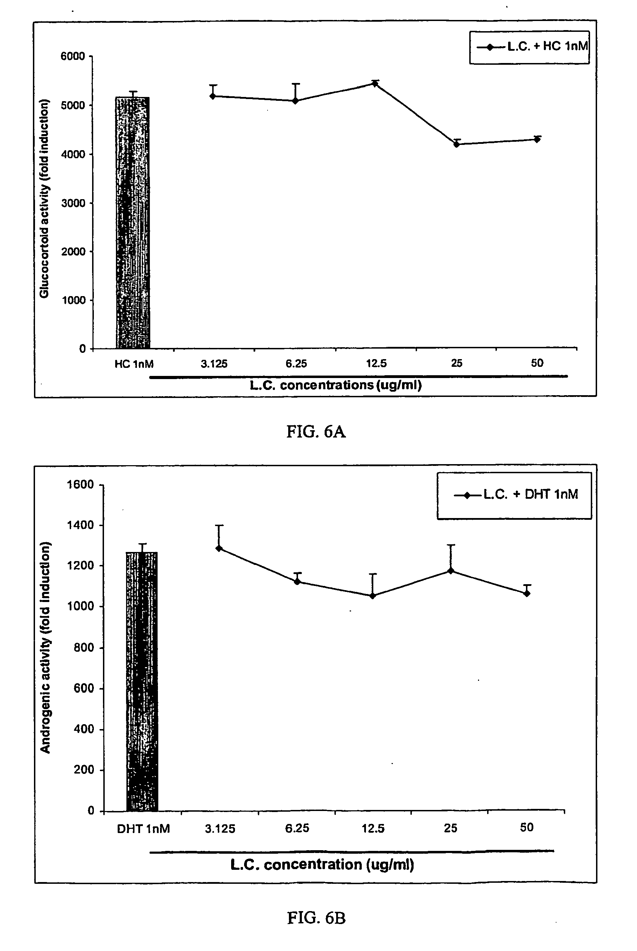

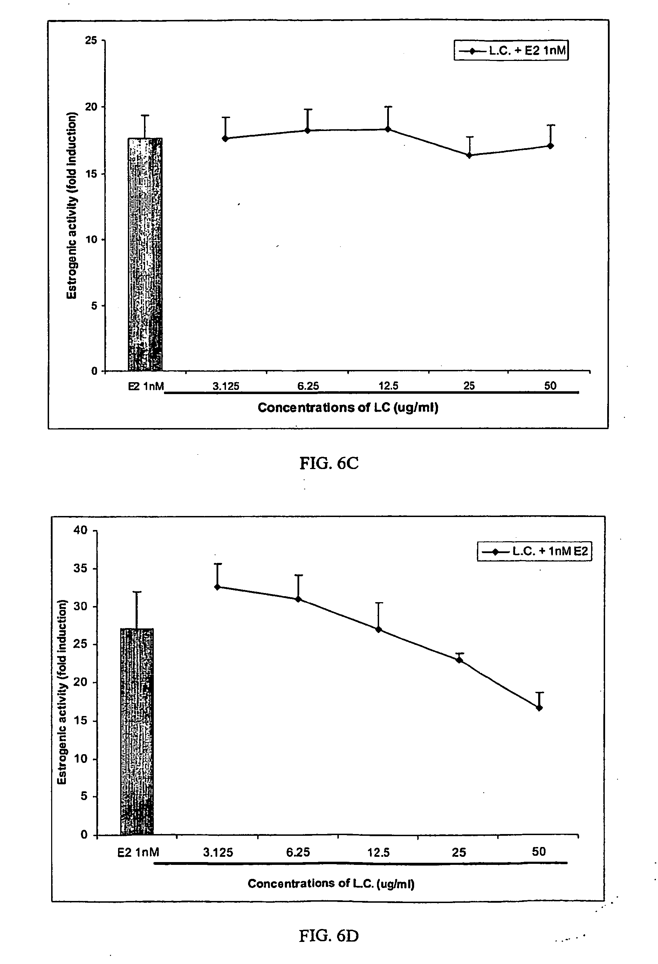

Figure 6 (A-D) shows the effects of ***L.C.*** crude extract on GR, AR, ERα and ERβ activity in the presence of their natural ligands. The effect of increasing doses *of **L.C.*** crude extract on Hormone receptor activity was assessed as described in Figure 5. Luciferase activity was measured for GR (Figure 6A), AR (Figure 6B), ERg (Figure 6C) and ERβ (Figure 6D); in the presence of fixed doses of hydrocortisone (HC 1nM), dihydrotestoterone (DHT 1nM) and estradiol (E2 1nM) respectively. No significant additive, synergistic, or antagonistic activity was observed.

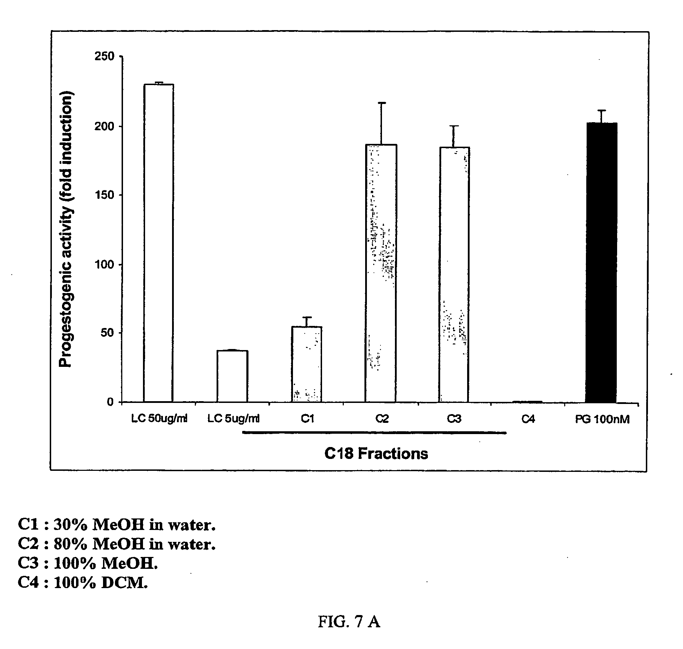

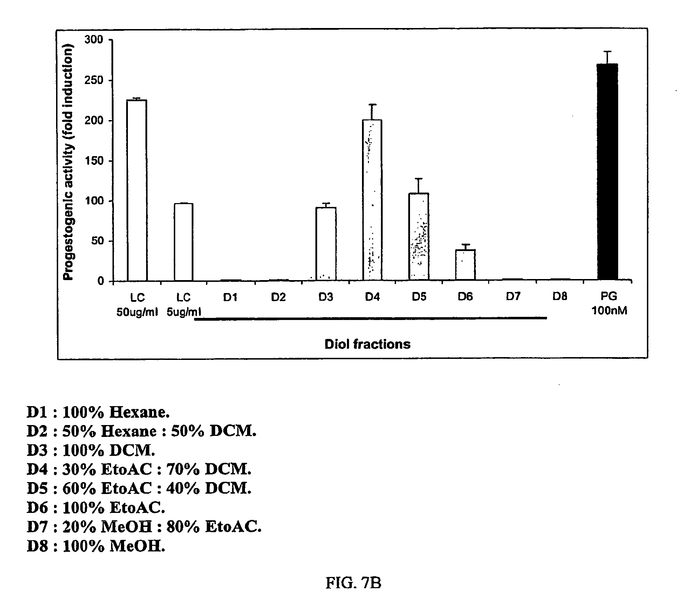

Figure 7 (A, B) shows the progestogenic activity of ***L.C.*** fractions following solid phase extraction. ***L.C.*** crude extract was applied to A) C18 reverse phase, and B) Diol flash columns. C18 or Diol powder was obtained commercially and packed into glass columns. ***L.C.*** (50mg/ml) was applied to the columns. Elution was performed in the order (C1 to C4) for C18 columns, and in the order (D1 to D8) for the Diol columns, using the solvents indicated. The fractions were collected separately in glass tubes, and dried down and reconstituted. Progestogenic activity was measured and expressed as fold-increase in luciferase activity compared with control cells exposed to vehicle only. Replicate wells exposed to ***L.C.*** extract (5 & 50µg/ml) alone, or PG 100nM alone, served as positive controls. All data points were in triplicate. Error bars represent SEM.

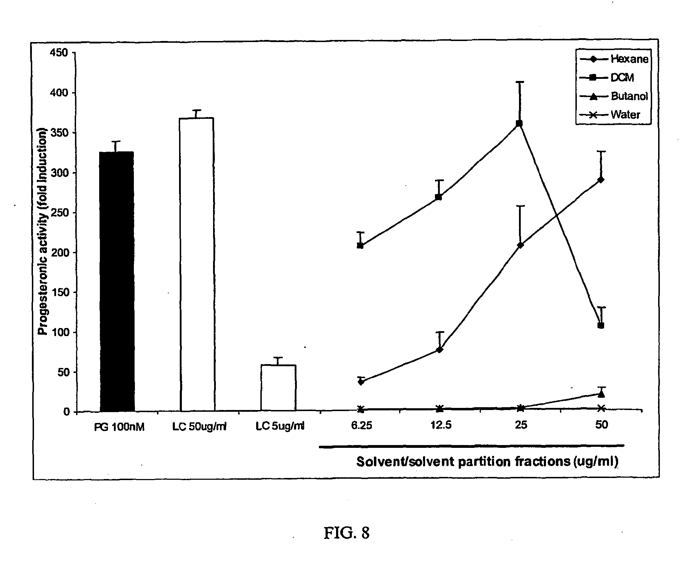

Figure 8 shows the progestogenic activity of ***L.C.*** fractions after solvent/solvent partition. ***L.C.*** crude extract in 100% ethanol (50mg/ml) was reconstituted to a mixture of 9:1 with water. 100% hexane was then added and partitioned the hexane phase was removed to form the first fraction. The next partition was conducted with the ethanol fraction in a mixture of 3:2, equal volume of DCM was added. This formed the second fraction. Following which an equal volume of Butanol was used for the final partition. Progestogenic activity was measured and expressed as fold-increase in luciferase activity compared with control cells exposed to vehicle only. Replicate wells exposed to ***L.C.*** extract (5 & 50µg/ml) alone, or PG 100nM alone, served as positive controls. All data points were in triplicate. Error bars represent SEM.

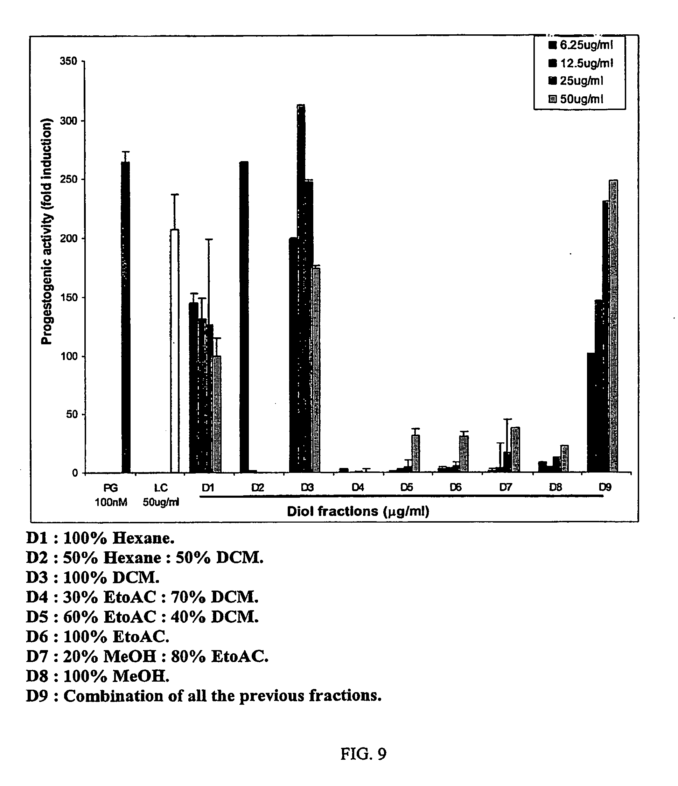

Figure 9 shows the progestogenic activity of Diol elution fractions following solvent/solvent extractions. The DCM fraction from solvent/solvent partition, shown in Figure 8, was subjected to Diol solid phase extraction and eluted with solvents (D1 to D8) as indicated. The fractions were collected separately in pre-weighed glass tubes, dried down, reweighed and reconstituted. Progestogenic activity of indicated doses of each Diol fraction was measured and expressed as fold-increase in luciferase activity compared with control cells exposed to vehicle only. Replicate wells were exposed to ***L.C.*** extract (50µg/ml) alone, or PG 100nM alone, or the reconstituted extract combining all Diol fractions (D9). All data points were in duplicate or triplicate. Error bars represent SEM.

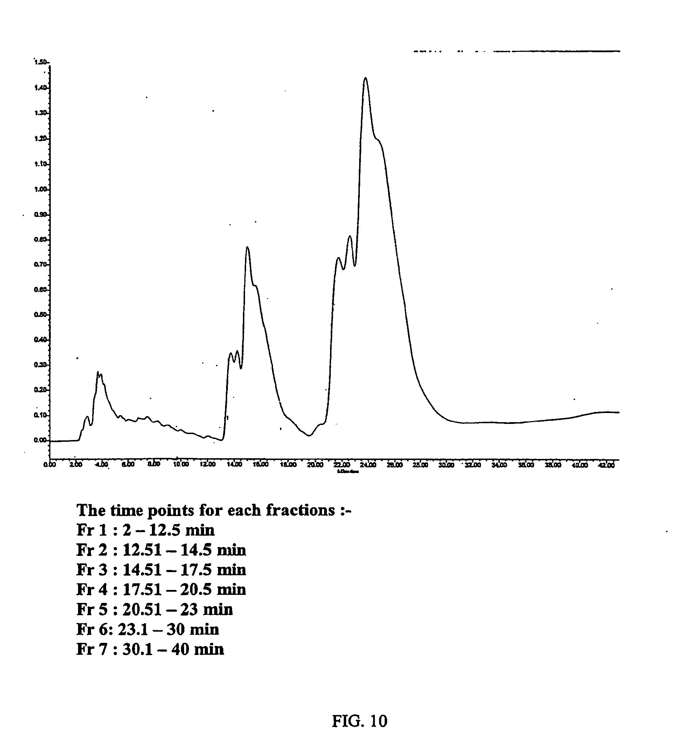

Figure 10 is a HPLC chromatogram of a bioactive Diol ***L.C.*** fraction. Diol fraction D1, eluted with 100%Hexane in Example 9, was further fractionated using HPLC. The HPLC column used was ThermoQuest Hypurity Elite C18 (5µm 150mm X 10mm). Isocratic conditions of 45%ACN:55%H₂O over a period of 40 minutes were used to effect the separation. This typical HPLC chromatogram of a purified ***L.C.*** extract, generated using a wavelength (λ) of 210nm, displayed 7 characteristic peaks within the elution periods Fr 1-Fr 7 as indicated.

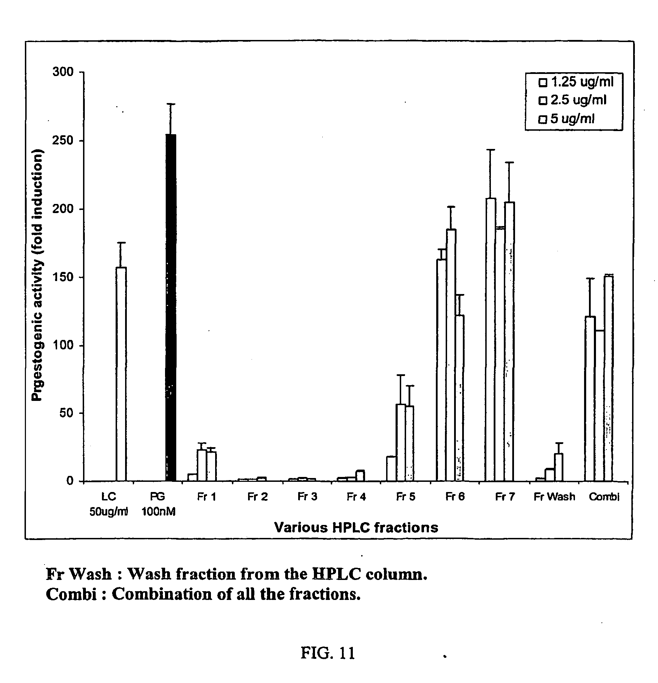

Figure 11 shows the progestogenic activity of the HPLC fractions. The 7 fractions with elution periods Fr 1-Fr 7 in Example 10 were dried down and re-constituted into 3 concentrations (5, 2.5 and 1.25µg/ml). These were then tested for the progestogenic activity with the assay described in Figure 1. Fraction 6 (Fr 6) demonstrated the most potent activity from the bioassay. Replicate wells were exposed to ***L.C.*** extract (50µg/ml) alone, or PG 100nM alone, or the reconstituted extract combining all Diol fractions (Combi). Maximum activity was observed in Fr 6 and Fr 7. In contrast, the wash after Fr 7 was relatively inactive. All data points were in duplicate or triplicate. Error bars represent SEM.

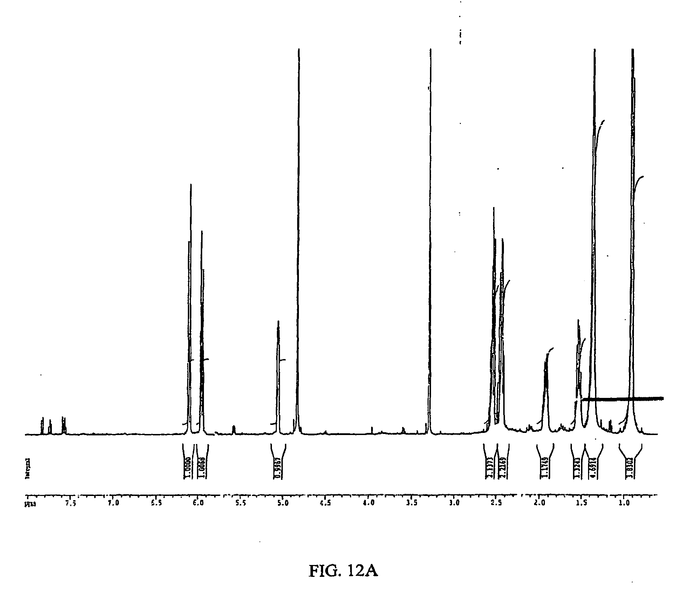

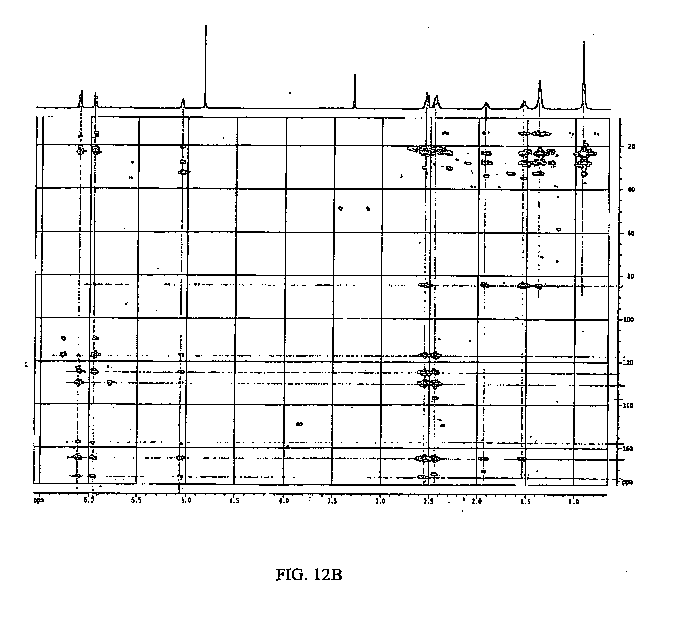

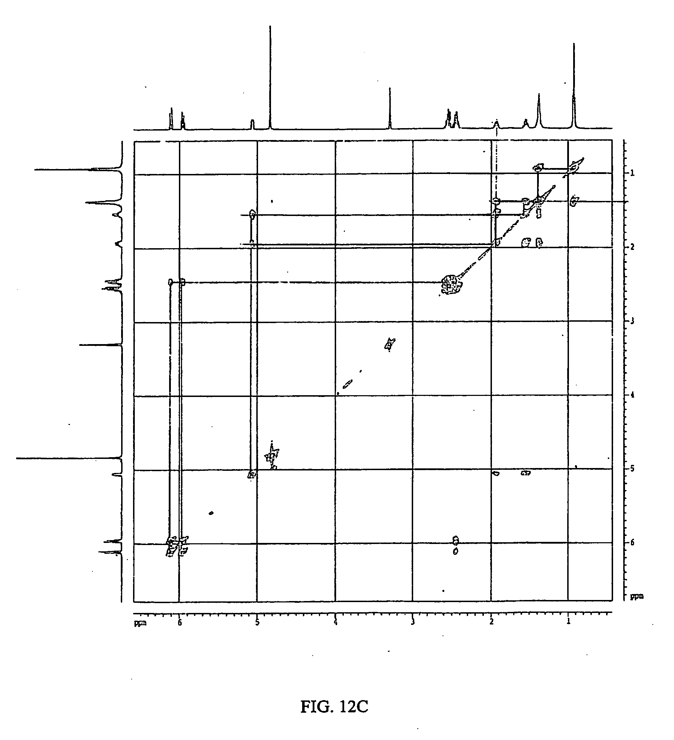

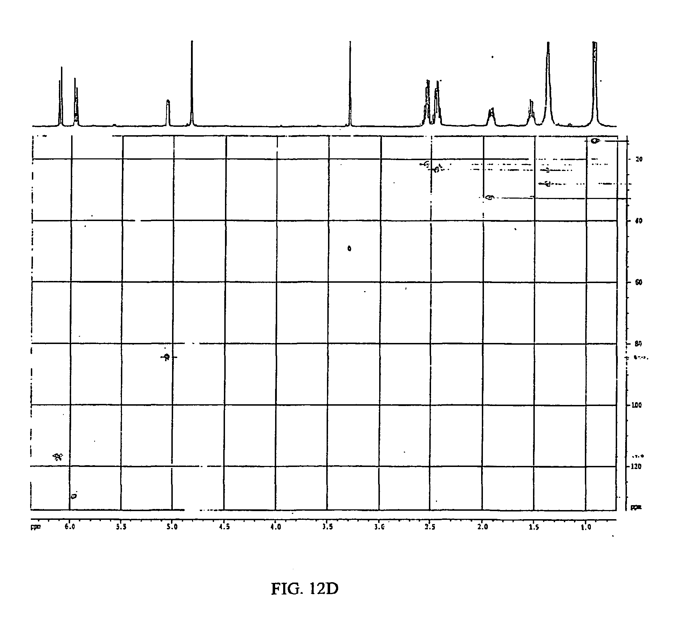

Figure 12(A-D) is the nuclear magnetic resonance (NMR) analyses of compounds in HPLC Fraction 6. Reconstituted Fr 6 from the preparative HPLC described in Example 10 was subjected to NMR analyses to determine the structure of their constituents. A) 1-D NMR, B) 2-D NMR, C) ¹H - ¹H COSY, D) ¹H -¹³C gHSQC.

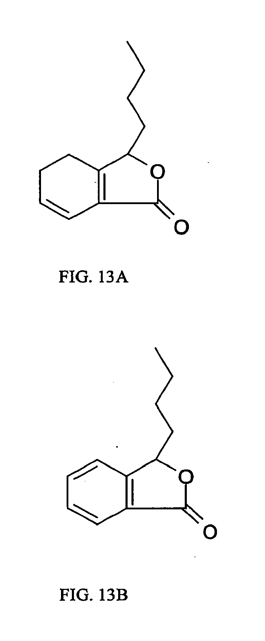

Figure 13(A-B) shows the structure of the constituents of HPLC Fraction 6. (A) 3-butyl-4,5-dihydrophthalide and (B) 3-butyl-phthalide.

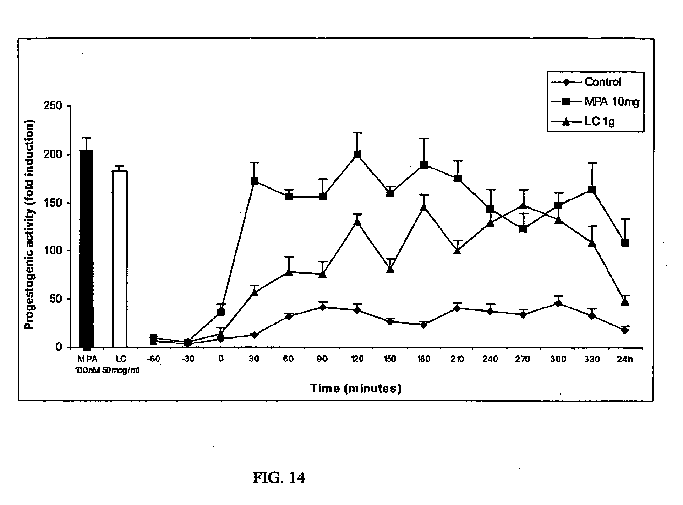

Figure 14 shows the progestogenic activity of L.C. in serum following subcutaneous administration. One ml. of crude ethanolic ***L.C.*** extract (1g), MPA (10mg) and control [1,2-propanediol:PEG(400):Ethanol (1:1:1)] was subcutaneously administered to male Sprague-Dawley rats at 0 minutes. Six rats were used for each arm of the experiment. Serum samples were collected from the tail vein at indicated time points, before and after ***L.C.*** injection. Bioactive progestogens were extracted from serum and tested for progestogenic activity using the novel receptor-based bioassay of the invention. Each data point is the Mean ±SEM of samples obtained from 6 different rats. Bars indicate progestogenic activity of original MPA and ***L.C.*** extract *in-vitro.* Progestogenic activity is expressed as fold increase compared to cells exposed to vehicle only.

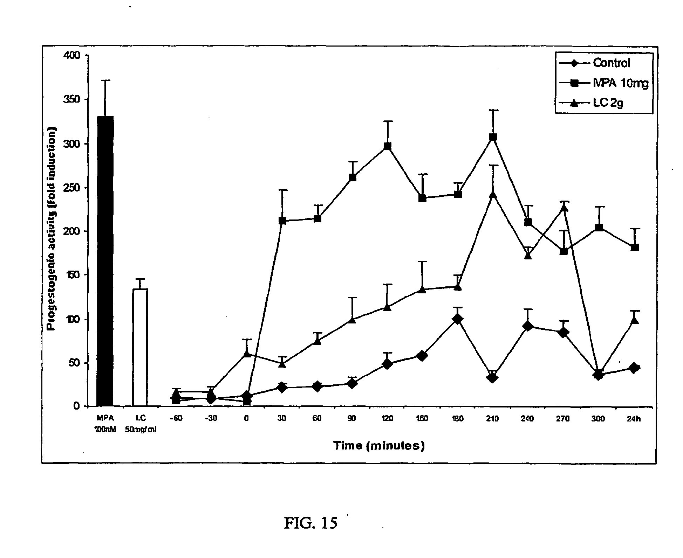

Figure 15 shows the progestogenic activity of ***L.C.*** in serum following ORAL administration. Sprague-Dawley rats were fasted overnight. Two mls *of **L.C.*** (2g), MPA (10mg) and vehicle (60% Ethanol) were orally fed to male rats using gavage tubes. Serum samples were collected from the tail vein at indicated time points, before and after ***L.C.*** administration. Bioactive progestogens were extracted from serum and tested for progestogenic activity using our receptor-based bioassay. Each data point is the Mean ±SEM of samples obtained from 6 different rats. Bars indicate progestogenic activity of original MPA and ***L.C*.** extract *in-vitro.* Progestogenic activity is expressed as fold increase compared to cells exposed to vehicle only.

### Detailed description

Different herbs reported to have "uterotonic activity" were identified and screened for progestogenic activity using a cell-based reporter gene assay and have discovered two herbs with significant progestogenic activity. Extracts from the *Rhizoma Chuanxiong,* the dried rhizome of *Ligusticum Chuanxiong* Hort., were able to specifically activate the progesterone receptor but not the related androgen, estrogen or glucocorticoid receptors. Dose response studies in cells indicate that predicted therapeutic serum levels for *Rhizoma Chuanxiong* crude extract, prepared as by methods described are between 6.25µg/ml to 100µg/ml, and the effective oral dose would be between 31.25mg to 500mg. Extracts containing these phyto-progestogens from *Rhizoma Chuanxiong* are useful to treat health problems requiring progesterone therapy, supplementation or replacement including fetal support, menstrual disorders, amenorrhoea, menorrhagia, polycystic ovarian syndrome, pregnancy complications, endometriosis, contraception, menopause, endometrial hyperplasia and endometrial cancer. These phytoprogestogens alone, or in combinations with other compounds, are also useful for oral contraception, for hormonal replacement therapy, for treating endometriosis, neuroprotective and neuroregenerative agents in stroke and traumatic brain injuries. It was also observed that compound(s) co-eluting with two known phthalides, 3-butyl-4,5-dihydrophthalide and 3-butyl-phthalide, from *Rhizoma Chuanxiong* strongly activate the progesterone receptor. These compound(s), which co-elute with 3-butyl-4,5-dihydrophthalide and 3-butyl-phthalide or herbal extracts enriched therewith, are useful for hormone replacement therapy and the treatment of health problems requiring progestogenic supplementation or therapy as described above.

The present disclosure also provides novel methods for separating fractions of *Rhizoma Chuanxiong,* enriched for 3-butyl-4,5-dihydrophthalide, 3-butyl-phthalide and components and co-elutes, with strong progestogenic activity using solvent-solvent partitioning, solid-phase fractionations and high-performance liquid chromatography (HPLC). These methods can be used to standardize and fingerprint *Rhizoma Chuanxiong* extracts using solvent-solvent partitioning solid phase fractionations, HPLC, mass spectrometry (MS), and nuclear magnetic resonance imaging (NMR). Since the bioactive compounds co-elutes with 3-butyl-4,5-dihydrophthalide and 3-butyl-phthalide, these compounds can be used for the measurement and standardization of the bioactivity *Rhizoma Chuanxiong* and its' extracts. Novel methods have been devised to detect summated bioavailability and bioequivalence of multiple steroidogenic compounds in serum, following drug administration to humans and animal models. It has also been established that *Rhizoma Chuanxiong* extract is an effective progestogenic drug when administered both subcutaneously and orally to animal models.

### Validation of in-vitro reporter gene assay for progestogens

In the progestogen receptor assay of the invention, HeLa cells grown in 24-well microtiter plates were transiently co-transfected with two plasmids using lipofectamine. The first plasmid consisted of DNA encoding the full length human progesterone receptor (PR-B), and the second a progesterone reporter gene (PRE2-TATA-LUC) comprising a luciferase reporter gene driven by two copies of the progesterone response element from the aminotransferase gene. Cells were exposed to the ligands in RPMI 1640 medium, supplemented with 10% charcoal-stripped fetal calf serum, 2mM L-glutamine, 0.1mM non-essential amino acids and 1mM sodium pyruvate for 42-48 hours at 37°C in a 5% carbon dioxide incubator. Replicate wells were exposed to vehicle only (ethanol) and used as negative controls. After the period of incubation, cells were rinsed with PBS buffer, lysis buffer (Promega Corp) was added, and cell lysates collected for measurement of luciferase activity. Progestogenic activity of the ligands was expressed as fold-increase in luciferase activity compared with control cells exposed to vehicle only. All data points were in triplicate. To determine the sensitivity of progesterone receptor assay, the transfected cells were exposed to increasing doses of one natural (progesterone), and one synthetic (megesterol acetate) progestogen (Figure 1). Both progesterone and megesterol acetate were able to dose-dependently increase reporter gene activity at doses ranging from 0.1 nM onwards, reaching peak activity at 10µM, wherein a 300-fold increase in hormonal activity was observed over controls exposed to vehicle only. Thus the assay system of the invention provides an effective way to screen for nano-molar quantities of bioactive progestogens.

### Novel Uses of extracts from Rhizoma Ligusticum Chuanxiong (L.C.) for progesterone therapy.

As will be clear from Example 1 below, from among 13 herbs screened, extracts derived from *Rhizoma Ligusticum Chuanxiong (**L.C.**)* were identified as possessing strong progestogenic activity. The most potent were ***L.C.*** extracts which displayed progestogenic activity comparable to progesterone itself (Example 1). Since progesterone and its derivatives are commonly used for hormone replacement therapy and other progesterone deficient states; crude herbal extracts of *Rhizoma Ligusticum Chuanxiong* with defined progestogenic activity can be useful to treat health problems requiring progestogen therapy. Such conditions include menstrual disorders, amenorrhoea, menorrhagia, polycystic ovarian syndrome, pregnancy complications, endometriosis, contraception, menopause, endometrial hyperplasia and endometrial cancer. The extracts in combinations with other compounds are also useful for oral contraception, for hormonal replacement therapy, for treating endometriosis, as neuroprotective and neuroregenerative agents in stroke and traumatic brain injuries.

### Novel methods to efficiently extract compounds from Rhizoma Ligusticum Chuanxiong that specifically activate the progesterone receptor.

Various combinations of ethanol/water, methanol/water and water alone, were tested to determine the best solvent for extracting progestogenic compounds from ***L.C.*** Extraction with 100% ethanol proved to be the most effective method for producing a crude extract with the highest progestogenic activity, comparable to progesterone itself (100nM) (Table 2). In contrast, extraction with methanol resulted in activity that was only one-third of 100% ethanol. Extraction with water resulted in an *inactive* extract. The method of extraction comprising soaking of powdered ***L.C.*** in 100% ethanol for 5-7 days at 37°C, that results in ***L.C.*** crude extract with maximal progestogenic activity is a novel and non-traditional method. It was also observed that while crude ***L.C.*** alcoholic extract has strong progestogenic activity, it does not have estrogenic, glucocorticoid or androgenic activity. Thus it is clear that ***L.C.*** extract contains compounds that can specifically activate the progesterone receptor but not other closely-related members of the steroid receptor family. Moreover it has an additive effect on progesterone action but has no antagonistic effects on the action of progestogens, estrogens, glucocorticoids or androgens. ***L.C.*** extracts prepared in the above manner are therefore particularly useful in health conditions in which pure and specific progestogenic effects are required and where cross reaction with other steroid receptor are contraindicated.

### Predicted therapeutic serum levels and effective oral doses in women.

It is believed that the crude ***L.C.*** extract and progesterone act through the same molecular mechanisms since their activity are blocked by the progesterone antagonist RU486 in an identical manner (Example 4). Thus the amount of ***L.C.*** required to achieve therapeutic effects can be inferred by comparison with the biological activity of physiological levels of progesterone. The range of serum progesterone concentrations in the human luteal phase is 15 to 90nM, and these doses of progesterone elicit 150- to 200-fold increases in reporter gene activity (Figure 1). Comparable 150- to 200-fold increases in reporter gene activity can be obtained with 6.25µg/ml to 100µg/ml of ***L.C.*** (Figure 2), suggesting that these doses of ***L.C.*** represent therapeutic serum dose ranges.

Assuming 100% absorption and distribution to the total blood volume of 5000ml, the oral dose of ***L.C.*** crude extract required for therapeutic effect is predicted to be between 31.25mg to 500mg. These doses of ***L.C.*** crude extract can be administered orally to treat conditions requiring progesterone replacement or supplementation as discussed above.

### New methods to purify L.C. fractions that are highly enriched for progestogenic activity

The present disclosure also provides a novel method to obtain ***L.C.*** fractions that are enriched for progestogenic activity. Such bioactive fractions are obtained by performing solid phase extractions using reverse phase C18 or Diol matrices in glass columns (Example 7). Using reverse phase C18 matrix, progestogenic activity is mainly present in fractions eluted with 80% to 100% methanol. With dry-packed Diol columns, maximal activity was observed with fractions eluted with 30%EtoAc:70%DCK and lesser degrees of activation with the 100%DCM and 60%EtoAc:40%DCM elutes. Removal of unwanted tannins can be achieved by first partitioning these into the butanol and water fractions, using solvent/solvent partitioning (Example 8).

Further purification if desired can be achieved using preparative HPLC. In this method a ThermoQuest Hypurity Elite C18 column and a isocratic mobile phase of 45%ACN:55%water can be used. Under these conditions, purified fractions can be expected to exhibit typical HPLC chromatograms as demonstrated in Example 10. In one manifestation, crude extract is first put through solvent/solvent partition to remove tannins, and then eluted through Diol solid phase columns using polar solvents like Hexane and DCM (Example 9). The elute when subjected to HPLC, separates into highly purified subfractions with strong progestogenic activity (Example 10). The present invention also provides for reproducible novel methods to obtain ***L.C.*** extracts with defined purity, consistency and biological activity.

These highly purified ***L.C.*** extracts can be use to treat conditions requiring progesterone therapy, supplementation or replacement. Such conditions include menstrual disorders, amenorrhoea, menorrhagia, polycystic ovarian *syndrome,* pregnancy complications, endometriosis, contraception, menopause, endometrial hyperplasia and endometrial cancer. The extracts in combinations with other compounds are also useful for oral contraception, for hormonal replacement therapy, for treating endometriosis, as neuroprotective and neuroregenerative agents in stroke and traumatic brain injuries.

### New methods for quality control and standardization of L.C. extracts

A key problem in botanical drugs is the lack of methods to standardize and quality control raw materials, herbal decoctions, the manufacturing process and the finished herbal products. Discovery of progestogenic properties of ***L.C.*** herbal extract will enable a person skilled in the art to use this property in appropriate cell-based or other assays to measure potency, purity and quality of the raw herbs and other manufactured herbal products derived from it. For example, progestogenic effects of the herbs can be measured with progestogenic-reporter gene assays in cell lines, and with ligand-binding assays; among others.

It has also been observed that characteristic HPLC conditions can be used to fingerprint ***L.C.*** herbal extracts. In one manifestation, ***L.C.*** extracts after preliminary solvent partition and solid phase extraction, can be subjected to HPLC C18 analysis (Example 10) resulting in typical HPLC chromatograms with distinct subfractions (Figure 10). Since the biological activity of each subfraction has been documented, these characteristic HPLC patterns, especially peaks corresponding to the relative quantity and resolution of each subfraction, can be used to standardize ***L.C.*** herbal extract.

If a higher level of standardization is required, these HPLC fractions can be subjected to NMR analyses, whereby characteristic patterns and chemical shifts can be observed (Fig, 12A, B, C, D). Those skilled in the art will realize that these methods of purification of active compounds can be adapted for standardization of the biological effect of the ***L.C.*** extracts. The methods of the present invention also enable those skilled in the art to devise methods for standardization and quality control of ***L.C.*** extracts using bioassays, solid phase fractionation, HPLC and NMR fingerprinting.

### Discovery of the progestogenic effects of Senkyunolide (A) and/or 3-butyl-phthalide and/or minor components co-eluting with them.

Two known phthalides, 3-butyl-4,5-dihydrophthalide and 3-butyl-phthalide were purified, isolated and identified from ***L.C.**,* along with components which co-elute with them, as potent activators of the progesterone receptor. Those skilled in the art will realize that the descriptions contained herein provides novel methods for measurement and standardization of the bioactivity ***L.C.*** and its extracts based on the presence of 3-butyl-4,5-dihydrophthalide and 3-butyl-phthalide. 3-butyl-4,5-dihydrophthalide and 3-butyl-phthalide, or components which co-elute with them, or herbal extracts enriched for them, are also useful for hormone replacement therapy and the treatment of human or animal health problems requiring progestogenic supplementation or therapy. Products derived from 3-butyl-4,5-dihydrophthalide and 3-butyl-phthalide, or components which co-elutes with them, are also expected to exhibit progestogenic effects and therefore can be used for progesterone therapy

### Novel methods to detect summated activity of multiple steroidogenic compounds in serum following drug administration to animal models.

Novel methods to perform pharmacokinetic and/or bioequivalence studies in human or in animal models have also been provided herein. These depend on the use of simple, robust techniques to precipitate proteins and extract bioactive small molecules from serum following parenteral or oral administration to animal models. The summated bioactivity of these molecules can then be tested in the receptor-based bioassay of the invention. Total biological activity of these extracted small molecules when compared to reference pharmaceutical compounds, enables the determination of bioequivalence. The assay of the present disclosure is useful as a biomarker of progestogenic action that can predict actual physiological changes due to progesterone in animals and in the human. The assay of the disclosure can measure progestogenic activity accurately and rapidly in days, compared to 4-8 weeks for traditional animal models of progestogenic activity, like the pregnancy maintenance and endometrial transformation models. The present assay is of use in the pharmaceutical industry where a rapid and discriminating biomarker to predict of the efficacy potential steroid-active drugs *in-vivo* is required. Since many steroidal pharmaceutical compounds are coming off patent, generic drug manufacturers need to prove bioequivalence to regulatory authorities. The present assay has great utility in this regard as a rapid and relatively inexpensive way to determine the absorption, distribution, metabolism, bioavailability and bioequivalence of pure compounds or complex mixtures compared to reference pharmaceutical compounds.

### Proof of principle that L.C. extract is an effective progestogenic drug when administered both subcutaneously and orally

The data in Examples 12 and 13 prove that ***L.C.*** ethanolic extract is efficiently absorbed when administered orally, that it remained biologically active in serum after absorption in the intestinal tract, and after the first-pass effect in the liver. Because the effect of ***L.C.*** declined rapidly after 5 hours (Example 13), it is believed that a 6 hourly (4 times a day) oral dosing regime will be required to maintain high progesterone activity throughout the day. MPA is used at a dose of 50mg/weekly (7.1mg/day) for endometriosis which is roughly equivalent to the dose of MPA (10 mg) in our experiments. Since ***L.C.*** can achieve a therapeutic equivalence of MPA 10 mg if administered at a dose of 2gm 6 hourly, the preferred dosage for LC crude extract for therapeutic use in humans would be about 250mg to 5gm every 6 hours or 1gm to 30 gm per day. The use of purified extracts enriched for bioactive progestogens would reduce the dose further. It has also been established in animal models that the progestogenic herbal *LC* extract is effective when administered subcutaneously or orally. The maximum progestogenic effect of the extract of the invention is equivalent to that observed with the gold standard progestogenic drug, Depo-provera. LC at the above doses can therefore be used for the same indications as Depo-provera, and these include abnormal menstrual bleeding, endometriosis, contraception, menopausal vasomotor symptoms, endometrial and renal carcinoma, and breast cancer.

The present disclosure will now be described with reference to the following examples, which are illustrative and should not be construed as limiting the scope of the invention in any manner.

### Example 1: Screening of herbs for progestogenic activity

To begin the search for phyto-progestogens in Traditional Chinese herbal medicines, The Pharmacopoeia of the People's Republic of China (1997) was consulted and 13 herbs reported to have "uterotonic activity" were selected for screening. Separate portions of the different chosen herbs were extracted with respective different concentration of solvents: 70% and 100% ethanol and 70% and 100% methanol. This was carried out by first powdering the herbs, mixing with the solvent, and allowing the mixture to soak for 5-7 days at 37°C, after which the respective extracts in the supernatant were filtered with Whatman Grade I (11µm pore size) filter paper. The herbal residues were re-extracted a second time with the same solvent for 2-3 hours. Filtered extracts were combined and dried in a rotary evaporator. This process was repeated with different combinations of solvents, namely 50%, 70% and 100% ethanol in water, and 70% and 100% methanol in water for each of the 13 selected herbs. Dried extracts were weighed and re-suspended in 100% ethanol or methanol to a concentration 50mg/ml. Each herbal extract was screened for progestogenic activity in-*vitro* at a final concentration of 50µg/ml.

Of the 13 herbal extracts screened, *Rhizoma Ligusticum Chuanxiong* (***L.C.***) displayed the greatest progestogenic activity. The herb ***L.C.**,* extracted with a 100% ethanol, demonstrated a 323-fold higher activity compared to controls exposed to vehicle alone as shown in Table 1. This was about 80% of the progestogenic activity observed with progesterone (100nM). In comparison, the other 11 extracts did not activate the PR. Thus it was discovered for the first time that potent phyto-progestogens are present in extracts from the dried rhizome of ***L.C.**,* a traditional Chinese herb.

**Table 1: Progestogenic activity of selected herbal extracts using PR and PRE2-TATA-LUC reporter gene in HeLa cells**

| **Herbal Extracts** | **Progestogenic activity (Fold-induction compared to controls)** | **Percentage activity compared to 100nM Progesterone (%)** |
|---|---|---|
| **Rhizoma Ligusticum Chuanxiong** | **323.39** | **79.86** |
| *Fructus Aurantii* | 1.81 | 1 |
| *Radix Glycyrrhizae* | 1.77 | 1 |
| *Rhizoma Corydalis* | 1.15 | 0.36 |
| *Rhizoma Atractylodis Marcocephalae* | 0.52 | 0.34 |
| *Radix Astragali* | 1.62 | 0.34 |
| *Radix Achyranthis Bidentatae* | 0.94 | 0.29 |
| *Herba Schizonepetae* | 1.31 | 0.27 |
| *Rhizoma seu Radix Notopterygii* | 0.49 | 0.27 |
| *Radix Peaoniae Alba* | 0.43 | 0.22 |
| *Concha Margaritifera Usta* | 0.39 | 0.12 |
| *Cortex Magnoliae Officinalis* | 0.27 | 0.05 |

### Example 2: Method for extraction of progestogenic compound(s) from L.C.

Various combinations of ethanol/water, methanol/water and water alone were tested to determine the best solvent for extracting progestogenic compounds from ***L.C.*** under conditions described in example 1. Extraction with a 100% of ethanol was the most effective method for producing a crude extract with the highest progestogenic activity, comparable to that of progesterone itself (100nM) (Table 2). In contrast, extraction with 100% methanol resulted in activity that was only one-third of 100% of ethanol. Extraction with water resulted in an inactive extract. This established a non-traditional method for extraction using 100% ethanol and repeated soaking for 5-7 days at 37°C that resulted in a ***L.C.*** crude extract with maximal progestogenic activity. In all subsequent experiments, extraction was performed with this method.

**Table 2: Progestogenic activity L.C. extracted with indicated solvents with the use of PR and PRE2-TATA-LUC reporter gene in HeLa cells.**

| *L.C.* crude extracts | **Progestogenic activity (% to PG 100nM)** |
|---|---|
| 70% Ethanol | 48.17 |
| 100% Ethanol | 79.86 |
| 100% Methanol | 23.39 |
| 100% Water | 0.53 |

### Example 3: Effect of L.C. crude extract in comparison with progesterone and predicted therapeutic dose ranges

The same methods as in Example 1 and 2 were adopted. The progestogenic activity of ***L.C.*** crude extract was compared to a physiological concentration of PG (100nM) (Figure 2). Doses of ***L.C.*** from 6.25µg/ml to 100µg/ml resulted in a dose-dependent increase in progestogenic activity. The maximal activity of ***L.C.*** crude extract, at 50µg/ml, was equivalent to that observed for 100nM progesterone. Since the maximum serum concentration of progesterone in women is 90nM, it is predicted that a serum L.C. level of 50µg/ml would be sufficient to achieve maximal therapeutic progestogenic effect. The physiological range of progesterone concentrations in the human luteal phase is 15 to 90nM, and these doses of progesterone elicit 150- to 200-fold increase in reporter gene activity (Figure 1). Comparable 150- to 200-fold increases in reporter gene activity are obtained with 6.25µg/ml to 100µg/ml of ***L.C.*** (Figure 2), suggesting that these doses of ***L.C.*** represent therapeutic serum dose ranges. Assuming 100% absorption and serum distribution to the total blood volume of 5000ml, the oral dose of ***L.C.*** crude extract required for therapeutic effect is predicted to be between 31.25mg to 500mg.

### Example 4: Inhibition of L.C. and progesterone action by the progesterone receptor antagonist, RU486, and possible therapeutic uses of L.C.

The same methods as in Example 1 and 2 were adopted. To determine the mechanism whereby ***L.C.*** activates the progesterone receptor, the effect of RU486 (a specific progesterone receptor antagonist) on ***L.C.*** activity was examined. The progestogenic effect of ***L.C.*** was dose-dependently inhibited by RU486, in a manner similar to that observed for progesterone itself (Figure 3). Thus doses of RU486 ≥0.01nM were able to completely inhibit the activity of fixed concentrations of both ***L.C.*** crude extract and progesterone. Since RU 486 acts by binding competitively to the ligand-binding pocket of the PR LBD, our data confirm that components in ***L.C.*** activate PR by specifically binding to the PR LBD in a manner similar to that for progesterone itself. Given that ***L.C*.** extract and progesterone act through similar mechanisms, ***L.C.*** herbal extract can be used for health problems for which progesterone replacement or supplementation is therapeutically indicated. Such conditions include menstrual disorders, amenorrhoea, menorrhagia, polycystic ovarian syndrome, pregnancy complications, endometriosis, contraception, menopause, endometrial hyperplasia and endometrial cancer. The extracts or phyto-progestogens from ***L.C.**,* alone, or in combinations with other compounds, are also useful for oral contraception, for hormonal replacement therapy, for treating endometriosis, neuro-protective and neuro-regenerative agents in stroke and traumatic brain injuries.

### Example 5: Additive effect of L.C. on progesterone activity

The same methods as in Example 1 and 2 were adopted. The presence of ***L.C.*** did not block the activity of progesterone. Addition of increasing doses of PG to a fixed concentration (50µg/ml.) of ***L.C.*** produced an additive increase in progestogenic activity (Figure 4) suggesting that ***L.C.*** can be used to boost the effects of endogenous progesterone.

### Example 6: Absence of agonistic and antagonistic activity on other steroid receptors, GR, AR, ER α and ER β.

The same methods as in Example 1 and 2 were adopted. Although ***L.C.*** exhibits progestogenic activity in a dose-dependent manner, doses of ***L.C.*** from 3.125µg/ml to 50µg/ml did not result in any activation of GR, AR, ERα and ERβ reporter gene systems (Figure 5). Similarly, the introduction of increasing concentrations of *L. C* crude extract did not elicit significant synergistic or antagonistic effect on the GR, AR, ERα and ERβ reporter gene systems in the presence of HC (1nM), DHT (1nM), and E2 (1nM) (Figures 6 A, B, C and D) respectively. The absence of cross-receptor activation with closely-related steroid receptors suggests that the action of ***L.C.*** was highly specific to the progesterone receptor.

### Example 7: Solid phase fractionation of L.C. with the use of C18 and Diol columns.

To identify the bioactive compounds in ***L.C.*** crude extract, bioassay-guided fractionation was performed. Preliminary isolation was performed with solid phase C18 and Diol columns.

Reverse phase C18 powder was packed into glass columns, after which 4 mls of ***L.C.*** (50mg/ml) was applied. Elution of ***L.C.*** fractions was performed with solvents of decreasing polarity in the following order: 30% MeOH, 80% MeOH, 100% MeOH and finally DCM. The fractions were collected separately dried down, reconstituted in ethanol and their bioactivity measured. Progestogenic activity was mainly present in fractions eluted with 80 to 100% methanol in water (Figure 7A). Smaller amounts were present in the 30% methanol fraction. In contrast, negligible activity was observed with 100% DCM, the most hydrophobic solvent.

Fractionation was also performed with Diol, a silica matrix. Diol powder was added to ***L.C.*** (50mg/ml) extract and the mixture dry-packed on top of a Diol column. Elution was performed using a least polar solvent to the most polar solvent. The elution solvents used were, 100% Hexane (D1), 50% DCM: 50% Hexane (D2), 100% DCM (D3), 30% EtoAc : 70% DCM (D4), (D5), 100% EtoAc (D6), 20% MEOH :80% EtoAc (D7) and finally 100% MeOH (D8). Figure 7B shows the progestogenic activity of the fractions obtained with PR bioassay as described in Figure 1. Maximal activity was observed with D4 (30% EtoAc: 70% DCM) fraction, and lesser degrees of activation with D3 (100% DCM) and D5 (60% EtoAc: 40% DCM).

These studies suggest that the bioactive compounds were of intermediate polarity since they co-elute with solvents of mixed polarity in both the diol and reverse phase C18 matrices. Studies herein also suggest that Diol and reverse phase C18 columns used with solvents of intermediate polarity can be used to obtain ***L.C.*** fractions that are enriched for progestogenic compounds.

### Example 8: Solvent/solvent partition

To further define the conditions under which separation of progestogenic fractions can be obtained, solvent/solvent partition of ***L.C.*** extracts was performed. Crude ***L.C.*** extract in ethanol (360ml) was mixed with water (40ml) to give a 90% ethanol solution, into which 400ml of hexane was added. The hexane was removed and the ethanol:water ratio adjusted to 3:2 to obtain optimal partition of DCM/ethanol-water interface. An equi-volume of DCM was added to the mixture. After this separation, DCM layer was removed and butanol was added. The butanol layer was separated from the water layer. All 4 solvent fractions were dried down, weighed, and their constituents tested for the PR bioactivity. Under these conditions, the majority of the bioactivity resided in the DCM and hexane fractions (Figure 8). The most potent was the DCM fraction, where dose-dependent increase in PR activity was observed such that 25µg/ml of this fraction was equivalent to 100nM of progesterone. The hexane fraction also exhibited strong bioactivity and 50µg/ml of this fraction was equivalent to 100nM of progesterone. In comparison, the butanol and water fractions did not show any bioactivity. Since tannins (substances known to interfere with receptor-based assays) partition mainly to butanol and water fractions, this method is useful for removing these unwanted tannins from bioactive fractions. The method of solvent/solvent partition can therefore be used to obtain DCM and hexane ***L.C.*** fractions, enriched for progestogenic compounds, which are free of tannins.

### Example 9: Second separation step of L.C. after solvent partition, with the use of the Diol solid matrix.

As the DCM fraction obtained from solvent/solvent partition exhibited greatest activity, it was used for further bioassay-guided fractionation. The DCM fraction was subjected to Diol solid-phase extraction and eluted with solvents of increasing polarity as described in Example 7. The fractions were collected and analyzed for bioactivity. Fractions D1, D2 and D3, eluted with 100%Hexane, Hexane/DCM (1:1) and 100% DCM respectively, showed the most progestogenic activity (Figure 9). Thus the combination of solvent/solvent partition (described in Example 9 above) and Diol Solid Phase Extraction, using non-polar solvents like Hexane and DCM, is useful to further purify fractions from ***L.C.*** that are enriched for progestogenic compounds.

### Example 10: HPLC finger printing of the fraction D1 from the diol column.

To further separate the bioactive components of ***L.C.**,* the Diol fraction D1 (obtained as in Example 9) was subjected to preparative HPLC analyses using a ThermoQuest Hypurity Elite C18 column and a isocratic mobile phase of 45%ACN:55%water. Under these conditions, Diol Fraction D1 exhibited a typical HPLC chromatogram from which 7 distinct subfractions can be recognized (Figure 10). These subfractions (with elution times as noted in Figure 10) were dried, weighed and their progestogenic activities determined. Progestogenic activity was greatest in subfractions 6 and 7, eluting from 23.1 min to 40 min (Figure 11). Decreasing amounts of bioactivity was observed in subfractions 5 and 1, with elution periods of 20.51 to 23 min. and 2 to 12.5 min. respectively. In contrast minimal bioactivity was detected in subfractions 2 to 4 eluting from 12.51 to 20.5 min. Our data indicate that more than one compound in ***L.C.*** is responsible for the progestogenic activity of ***L.C.**,* since bioactive subfractions 1 and 5-6 are separated by inactive subfractions. The HPLC chromatogram as shown Figure 10 can be used as a standard for biological activity of ***L.C.*** Under these HPLC conditions, the subfractions 6 and 7 (eluting from 23.1 min to 40 min) can be expected to contain the most potent progestogenic compounds.

### Example 11: NMR finger printing of the fraction 6 obtained from HPLC fractionation.

To identify the bioactive compounds, the HPLC fraction with the highest yield (subfraction 6) was subjected to NMR analysis. A known phthalide, Senkyunolide (A) or 3-butyl-4,5-dihydrophthalide, was identified in subfraction 6, using ID- and 2D-NMR, ¹H-¹H COSY, ¹H-¹³C gHSQC spectral analyses (Figure 12A, B, C, D). Senkyunolide (A) has the molecular formula C₁₂H₁₆O₂ corresponding to five double-bond equivalents. The NMR data for Senkyunolide (A) revealed resonances consistent with an ester carbonyl carbon (¹³C 165.0) and a 3,4-disubstituted 1,3 cyclohexadiene (¹H δ 5.96, 6.10 and ¹³C 130.0, 117.0, 125.0 and 137.5). Evident were resonances consistent with a butyl group (¹H δ 1.93, 1.38, 1.38, 0.93 and ¹³C 33.0, 27.0, 24.0 and 14.5) and an oxymethine (¹³C 84.5 and ¹H δ 5.06). Analysis of gHMBC showed important correlations from ¹H δ 2.54 to ¹³C 165.0, 137.5, 130.0, 125.0 and 117.0 ppm and ¹H δ 5.06 to ¹³C 165.0, 130.0, 125.0, 117.0 and 33.0 and 27.0 ppm. This allowed the gross structure of the compound to be proposed as shown (Figure 13A). This data is consistent with the reported structure (Yu, 1983). Using similar analyses a related second compound, 3-butyl-phthalide (Figure 13B), was characterized in subfraction 6. Analyses of 1D-NMR data show that the principal constituent of subfraction 6 is Senkyunolide (A), constituting 95% of the dried extract by weight. 3-Butyl-phthalide, forming 2% of the extract is a minor constituent. Thus it is very likely that Senkyunolide (A) and/or 3-butyl-phthalide and/or minor co-elutes, are progestogenic and can be used to treat conditions requiring progesterone replacement therapy or supplementation. Furthermore, Senkyunolide (A) and/or 3-butyl-phthalide and/or minor co-elutes, can be used as markers for progestogenic bioactivity in ***L.C.*** extracts.

### Example 12: Detection of progestogenic activity in serum following drug administration to animal models.

L.C*.* extract was administered to rat models and its progestogenic effect in serum compared with vehicle (negative control) and a reference progestogenic drug, 6α-methyl-17α-hydroxy-progesterone acetate (Depo-provera, MPA). ***L.C.*** extract (1gm/ml), vehicle (1 ml) and MPA (10mg/ml) were injected subcutaneously to male Sprague-Dawley rats and peripheral blood (150µl) sampled at indicated time intervals for 24 hours from tail veins. Male rats were selected because of their low endogenous progesterone levels. Collected blood samples were stored at 4°C until processed. Whole blood was centrifuged at 4°C for 5 minutes and serum collected. Two parts of extraction solvent, consisting of acetonitrile:methanol (3:1), was added to one part of serum and the mixture vortexed for 30 sec to precipitate proteins. The precipitate was then removed by centrifugation at 10,000G for 5 min. The clear supernatant containing bioactive small molecules was stored at -20C for bioassay. Subsequently 6µl of this supernatant (equivalent to 2µl of serum from the rat) was used for the progestogenic bioassay. To increase the assay sensitivity, HeLa cells were pre-exposed to charcoal-stripped serum in phenol free RPMI for at least 7 days prior to performing the assay. As shown in Figure 14, progestogenic activity in serum rose to a peak within 30 min of subcutaneous injection of the reference drug MPA. The peak progestogenic activity observed with MPA was maintained for more than 24 hours and was about 5-fold higher than serum from rats administered vehicle only. This level of progestogenic activity is equivalent to the maximum bioactivity of MPA extract when tested *in-vitro* (black bar). Serum from rats injected with LC displayed a slower but still significant rise in progestogenic activity reaching a peak after 270 min. At its peak, LC activity was comparable to that observed with saturating doses of MPA *in-vitro* and in-vivo. High levels of progesterone activity was observed from 120 to 330 min after injection and declined to 31 % of peak levels after 24 hours. This proves the principle that the technology described in this example can accurately quantify the summated bioactivity of progestogenic compounds in serum following administration of progestogenic drugs to animal models.

### Example 13: Progestogenic activity of LC in a rat model following oral administration.

To determine the pharmacokinetics and bioequivalence of LC*,* we measured the progestogenic effect of rat serum sampled at defined time points after oral doses of *LC* (2g/2ml), MPA (10mg/2ml), and vehicle only (2 ml 60% ethanol). Serum samples were extracted and progesterone effect measured as in Example 12. Rats fed MPA exhibited a rapid and highly significant rise in serum progestogenic action reaching a peak after 120 min (Figure 15). High levels ofprogestogenic effect were maintained throughout the 24 hour of sampling. In contrast, rats administered vehicle only exhibited baseline progesterone activity that was about 5-fold lower than that observed with MPA. Rats fed LC showed a steady rise in progesterone activity reaching a peak after 210 min. This peak was maintained for an hour before-dropping rapidly to baseline after 300 min. Rats fed either MPA or LC displayed peak progesterone activity that was comparable to that observed with saturating doses of progesterone *in-vitro.* Based on these pharmacokinetic/pharmacodynamic profiles, we conclude that oral doses of LC can result in maximal progesterone activity in serum. In our model, significant effects were observed after 60 min, peaks at 210 min and returns to baseline after 270 min following LC administration. These data prove that LC ethanolic extract is efficiently absorbed when administered orally and that it remains biologically active in serum after absorption in the intestinal tract and after the first-pass effect in the liver. Because the effect of LC declines rapidly after 5 hours, we conclude that a 6 hourly (4 times a day) oral dosing regime might be required to maintain high progesterone activity throughout the day. MPA is used at a dose of 50mg/weekly (7.1mg/day) for endometriosis which is roughly equivalent to the dose of MPA (10 mg) in our experiments. Since LC can achieve a therapeutic equivalence of MPA 10 mg if administered at a dose of 2gm 6 hourly, the preferred dosage for LC crude extract for therapeutic use in humans would be about 250mg to 5gm every 6 hours, or 1gm to 30 gm per day. The use of purified extracts enriched for bioactive progestogens would reduce the dose further.

### Advantages disclosed

A) Discovery of extracts from the *Rhizoma Chuanxiong,* the dried rhizome of *Ligusticum chuanxiong* Hort., a traditional Chinese herb, which specifically activates the progesterone receptor (phyto-progestogens).
B) Novel methods to efficiently extract progestogenic compounds from *Rhizoma Chuanxiong.*
C) Prediction of therapeutic serum levels for *Rhizoma Chuanxiong* crude extract, prepared as by methods described in (B), as being between 6.25µg/ml to 100µg/ml, and the effective oral dose being between 31.25mg to 500mg.
D) Use of extracts, containing these phyto-progestogens from *Rhizoma Chuanxiong,* to treat health problems requiring progesterone therapy, supplementation or replacement including fetal support, menstrual disorders, amenorrhoea, menorrhagia, polycystic ovarian syndrome, pregnancy complications, endometriosis, contraception, menopause, endometrial hyperplasia and endometrial cancer.
E) Use of extracts, containing these phyto-progestogens from *Rhizoma Chuanxiong,* alone, or in combinations with other compounds, for oral contraception, for hormonal replacement therapy, for treating endometriosis, neuroprotective and neuroregenerative agents in stroke and traumatic brain injuries.
F) Discovery of compound(s) co-eluting with two known phthalides, 3-butyl-4,5-dihydrophthalide and 3-butyl-phthalide, from *Rhizoma Chuanxiong* which strongly activates the progesterone receptor.
G) Use of compound(s), which co-elutes with 3-butyl-4,5-dihydrophthalide and 3-butyl-phthalide or herbal extracts enriched for them, for hormone replacement therapy and the treatment of health problems requiring progestogenic supplementation or therapy as described in (B) and/or (C).
H) New and simple methods to separate fractions of *Rhizoma Chuanxiong,* enriched for 3-butyl-4,5-dihydrophthalide, 3-butyl-phthalide and components and co-eluates, with strong progestogenic activity using solvent-solvent partitioning, solid-phase fractionations and high-performance liquid chromatography (HPLC).
I) Novel methods for standardization and fingerprinting of *Rhizoma Chuanxiong* extracts using solvent-solvent partitioning solid phase fractionations, HPLC, mass spectrometry (MS), and nuclear magnetic resonance imaging (NMR).
J) Novel methods for measurement and standardization of the bioactivity *Rhizoma Chuanxiong* and its extracts based on the presence of 3-butyl-4,5-dihydrophthalide and 3-butyl-phthalide.
K) Novel methods to detect summated bioavailability and bioequivalence of multiple steroidogenic compounds in serum following drug administration to humans and animal models.
L) First proof of the principle that *Rhizoma Chuanxiong* extract is an effective progestogenic drug when administered both subcutaneously and orally to animal models.

### BIBLIOGRAPHY

Bensky D., Gamble A. 1993. Chinese Herbal Medicine: Materia Medica, Revised Edition. Seattle, WA: Eastland Press.
Bouguet W., Ruff M., Chambon P., Grouemeyer H., Morea S. 1995. Crystal structure of the ligand binding domain of the human nuclear receptor RXR-alpha. Nature 375:377-382.
Carson-jurica M.A., Schrader W.T., O'malley B.W. 1990. Steroid receptor family structure and functions. Endocr. Rev. 11:201-220.
Chen D.R. 1992a. Comparative study of chuanxiong and dextran 40 in the treatment of acute cerebral infarction. Zhongguo Zhong Xi Yi Jie He Za Zhi. 12:71-3, 67.
Chen D.R. 1992b Clinical and experimental study of Ligusticum wallichii and aspirin in the treatment of transient ischemic attack. Zhongguo Zhong Xi Yi Jie He Za Zhi. 12:672-674.
Chen K.J., Chen K. 1992. Ischemic stroke treated with Ligusticum chuanxiong. Chin. Med. J. 105:870-3
Clarke C.L., Sutherland R.L. 1990. Progestin regulation of cellular proliferation. Endocrine. Rev. 11-266-300.
Hong YH. 1986. Oriental Materia Medica: A Concise Guide. Long Beach, CA: Oriental Healing Arts Institute.
Ito A., Imada K., Takashi S., Kubo T., Matsushima K., Mori Y., 1994. Suppression of interleukin 8 production by progesterone in rabbit uterine cervix. Biochem. J. 301:183-6.
Ko W.C., Chang C.Y., Sheu J.R., Tzeng S.H., Chen C.M. 1998. Effect of butylidenephthalide on calcium mobilization in isolated rat aorta. J. Pharm. Pharmacol. 50:1365-9.
Kobayashi M, Fujita M, Mitsuhashi H. 1984. Components of Cnidium officinale Makino: occurrence of pregnenolone, coniferyl ferulate, and hydroxyphthalides. Chem. Pharm. Bull. 32(9):3770-3.
Kurihara K, Tanabe K, Yamamoto Y, Shinei R, Ajito K, Okonogi T. 1999. Synthesis and structure-activity relationships of new non-steroidal progesterone receptor ligands. Bioorg. Med. Chem. Lett. 9:1837-42.
Lehman, Manfred, Schoellkopf, Klaus, Strehlke, Peter, Heinrich, Nikolaus, Fritzemeier, Karl-Heinrich, Krattenmacher, Rolf, Muhn, Hans-Peter. 2001. Nonsteroidal gestagens. USP 6,245,804.
Liu S. 1992. Effects of Ligustricum Wallichii on acute nephrotoxicity induced by cyclosporine A in rats. Zhonghua Yi Xue Za Zhi. 72:345-7, 382.
Liu Z, Shi Y. 1990 Effects of Ligusticum wallichii on the plasma and CSF levels of dynorphin Ail - 1 3 in rabbits under acute experimental cerebral ischemia. Zhong Xi Yi Jie He Za Zhi. 10:160-1, 163, 133.
Liu Z. 1991. Effects of Ligusticum wallichii on the plasma levels of beta-thromboglobulin, platelet factor 4, thromboxane B2 and 6-keto-PGF1 alpha in patients with acute cerebral infarction. Zhong Xi Yi Jie He Za Zhi. 11:711-3, 707.
Lydon J.P., DeMayo F.J., Funk C.R., Mani S.K., Hughes A.R., Montgomery C.A. Jr, Shyamala G., Conneely O.M., O'Malley B.W. 1995. Mice lacking progesterone receptor exhibit pleiotropic reproductive abnormalities. Genes. Dev. 9(18):2266-78.
Masayasu K, et al. 1989. Antiarteriosclerotic. JP 1207233.
Mimura Y., Kobayashi S., Naitoh T., Kimura L, Kimura M. 1995.The structure-activity relationship between synthetic butylidenephthalide derivatives regarding the competence and progression of inhibition in primary cultures proliferation of mouse aorta smooth muscle cells. Biol. Pharm. Bull. 18(9):1203-6.
Misrahi M., Venencie P.Y., Saugier-Veber P., Sar S., Denssen P., Milgrom E. 1993. Structure of the human progesterone receptor gene. Biochim. Biophys. Acta 1216:289-292.
Naito T., Katsuhara T., Niitsu K., Ikeya Y., Okada M., Mitsuhashi H. 1992. Two phthalides from ligusticum chuan xiong. Phytochemistry 31; 639-642.
Naito T., Kubota K., Shimoda Y., Sato T., Ikeya Y., Okada M., Maruno M. 1995. Effects of the constituents of a crude chinese drug, ligustici Chuan xiong Rhizoma, on vasoconstriction and blood viscosity. Nat. Med. 49,288-92.
Nakazawa K., Fujimori K., Inoue K., Sekita S., Takanaka A. 1989. Effects of extract from a herbal drug, cnidium rhizome (senkyu), on contraction, heart rates and membrane potentials of isolated guinea pig atria. Yakugaku Zasshi. 109:662-71.
Namba T., Sekiya K., Kadota S., Hattori M., Katayama K., Koizumi T. 1992. Studies on the baths with crude drug: the effects of Senkyu extract as skin penetration enhancer. Yakugaku Zasshi. 112:63 8-44.
Nuclear Receptor Nomenclature Committee. 1999. A unified nomenclature system for the nuclear receptor superfamily. Cell. 97:161-3.
Ozaki Y. 1992. Antiinflammatory effect of tetramethylpyrazine and ferulic acid. Chef. Pharm. Bull. 40:954-956.
Palmer S., Campen C.A. Allan G.F., Rybczynski P., Haynes-Johnson D., Hutchins A., Kraft P., Kiddoe M., Lai M., Lombardi E., Pedersen P., Hodgen G., Combs D.W. 2000. Nonsteroidal progesterone receptor ligands with unprecedented receptor selectivity. J Steroid Biochem. Mol. Biol. 1;75(1):33-42.
Paris F, Servant N, Terouanne B, Balaguer P, Nicolas JC, Sultan C. 2002 A new recombinant cell bioassay for ultrasensitive determination of serum estrogenic bioactivity in children. J Clin Endocrinol Metab. 87:791-7.
Paris F, Servant N, Terouanne B, Sultan C. 2002 Evaluation of androgenic bioactivity in human serum by recombinant cell line: preliminary results. Mol Cell Endocrinol. Dec 30;198(1-2):123-9.
Rousseau-Merck M.F., Misrahi M., Losefelt H., Milgrom E., Berger R. 1987. Localization of the human progesterone receptor gene to chromosome 11q22 - 11q23. Human Genet. 77:280-282.
Sekiya K., Kadota S., Katayama K., Koizumi T., Namba T. 1997. Study on baths with crude drug. III : The effect of ligustici chuanxiong rhizoma extract on the percutaneous absorption of some natural compounds. Biol. Pharm. Bull. 20(9):983-7.
Sengupta J., Ghosh D. 2000. Role of progesterone on peri-implantation stage endometrium-embryo interaction in the primate. Steroids 65(10-11):753-62.
Shao C.R, Chen F.M., Tang Y.X. 1994. Clinical and experimental study on Ligusticum wallichii mixture in preventing and treating bronchial asthma. Zhongguo Zhong Xi Yi Jie He Za Zhi. 14(8):465-8.
Sinclair S. 1998. Chinese herbs: a clinical review of Astragalus, Ligusticum, and Schizandrae. Altern. Med Rev. 3:338-44
Skouby S.O. 2000. The rationale for a wider range of progestogens. Climacteric. Suppl 2:14-20.
Stein D.G. 2001. Brain damage, sex hormones and recovery: a new role for progesterone and estrogen? Trends Neurosci. 24(7):386-91.
The Pharmacopoeia of The People's Republic of China (English Edition 1997), Beijing, China. Chemical Industry Press.
Tibbetts T.A., Mendoza-Meneses M., O'Malley B.W., Conneely O.M. 1998. Mutual and intercompartmental regulation of estrogen and progesterone receptor expression in the mouse uterus. Biol. Reprod. 59:1143-52.
Tibbettes T.A., Conneely O.M., O'Malley B.W. 1999. Progesterone via its receptor antagonizes the pro-inflammatory activity of esterogen in the mouse uterus. Biol. Reprod. 60:1158-65.
Vermeer H, Hendriks-Stegeman BI, Van Den Brink CE, Van Der Saag PT, Van Der Burg B, Van Buul-Offers SC, Jansen M. 2003 A novel specific bioassay for the determination of glucocorticoid bioavailability in human serum. Clin Endocrinol (Oxf). 2003 Jul;59(1):49-55.
Yu S.R., Gao N.N., Li L.L., Wang Z.Y., Chen Y., Wang W.N. 1988a. The protective effect of 3-butyl phthalide on rat brain cells. Yao Xue Xue Bao. 23(9):656-61.
Yu S.R., Gao N.N., Li L.L., Wang Z.Y., Chen Y., Wang W.N. 1988b. Facilitated performance of learning and memory in rats by 3-n-butyl phthalide. Zhongguo Yao Li Xue Bao. 9(5):385-8.
Yu C. 1983. Preliminary study on the constituents of essential oil from ligusticum chuanxiong Hort. Beijing Yixueyuan Xuebao 15:2217-19.
Zhang J.L., He X.F., Zhou Z.H. 1996. HPLC determination of five constituents in plants of genus Ligusticum. Yao Xue Xue Bao. 31:622-5.
Zhi L., Tegley C.M., Marschke K.B., Mais D.E., Jones T.K. 1999. 5-Aryl-1,2,3,4-tetrahydrochromeno[3,4-f]quinolin-3-ones as a novel class of nonsteroidal progesterone receptor agonists: effect of A-ring modification. J. Med. Chem. 42:1466-72.
Zhong F., Yang L., Ji L., Hu S., Fu G. 1996. Studies on the essential oils in Ligusticum chuanxiong Hort. of different habitats and species. Zhongguo Zhong Yao Za Zhi. 21:147-51.

## Claims

1. An alcoholic extract of Rhizoma Ligusticum Chuanxiong **characterized in that** the extract is capable of eliciting progestogenic activity in a progestogen receptor assay for use in treating conditions requiring progesterone replacement or supplementation in a human or animal, wherein the condition is selected from the group consisting of progesterone replacement, progesterone supplementation, menstrual disorders, amenorrhoea, menorrhagia, polycystic ovarian syndrome, pregnancy complications, endometriosis, contraception, menopause, endometrial cancer, and endometrial hyperplasia wherein said extract comprises 3-butyl-4,5-dihydrophthalide and 3-butyl-phthalide.

2. An alcoholic extract as claimed in claim 1, wherein the 3-butyl-4,5-dihydrophthalide is present in the extract in an amount of about 95wt% and the 3-butyl-phthalide is present in the extract in an amount of about 2wt%.

3. An alcoholic extract of Rhizoma Ligusticum Chuanxiong **characterized in that** the extract is capable of eliciting progestogenic activity in a progestogen receptor assay, for use in treating stroke or brain injuries in a human or animal wherein said extract comprises 3-butyl-4,5-dihydrophthalide and 3-butyl-phthalide.

4. An alcoholic extract as claimed in claim 3, wherein the 3-butyl-4,5-dihydrophthalide is present in the extract in an amount of about 95wt% and the 3-butyl-phthalide is present In the extract in an amount of about 2wt%.

## Patentansprüche

1. Alkoholischer Extrakt aus Rhizoma Ligusticum Chuanxiong, **dadurch gekennzeichnet, daß** der Extrakt befähigt ist, progestogene Aktivität in einem Progestogenrezeptor-Assay hervorzurufen, zur Verwendung in der Behandlung von Zuständen, die Progesteronersatz oder -ergänzung in einem Menschen oder Tier erfordern, wobei der Zustand aus der Gruppe, bestehend aus Progesteronersatz, Progesteronergänzung, Menstruationsstörungen, Amenorrhoe, Menorrhagie, polycystischem Ovarialsyndrom, Schwangerschaftskomplikationen, Endometriose, Kontrazeption, Menopause, endometrialem Krebs und endometrialer Hyperplasie, ausgewählt ist, wobei der Extrakt 3-Butyl-4,5-dihydrophthalid und 3-Butylphthalid umfaßt.

2. Alkoholischer Extrakt, wie in Anspruch 1 beansprucht, wobei das 3-Butyl-4,5-dihydrophthalid in dem Extrakt in einer Menge von etwa 95 Gew-% vorhanden ist und das 3-Butylphthalid in dem Extrakt in einer Menge von etwa 2 Gew.-% vorhanden ist.

3. Alkoholischer Extrakt aus Rhizoma Ligusticum Chuanxiong, **dadurch gekennzeichnet, daß** der Extrakt befähigt ist, progestogene Aktivität in einem Progestogenrezeptor-Assay hervorzurufen, zur Verwendung in der Behandlung von Schlaganfall oder Hirnverletzungen in einem Menschen oder Tier, wobei der Extrakt 3-Butyl-4,5-dihydrophthalid und 3-Butylphthalid umfaßt.

4. Alkoholischer Extrakt, wie in Anspruch 3 beansprucht, wobei das 3-Butyl-4,5-dihydrophthalid in dem Extrakt in einer Menge von etwa 95 Gew.-% vorhanden ist und das 3-Butylphthalid in dem Extrakt in einer Menge von etwa 2 Gew.-% vorhanden ist.

## Revendications

1. Extrait alcoolique du Rhizome Ligusticum Chuanxiong, **caractérisé en ce que** l'extrait est capable de déclencher une activité progestogénique dans un dosage des récepteurs aux progestatifs, pour une utilisation dans le traitement d'états nécessitant une substitution ou supplémentation en progestérone chez un être humain ou un animal, dans lequel l'état est choisi dans le groupe constitué par la substitution en progestérone, la supplémentation en progestérone, les troubles menstruels, l'aménorrhée, la ménorragic, le syndrome des ovaires polykystiques, les complications de la grossesse, l'endométriose, la contraception, la ménopause, le cancer de l'endomètre et l'hyperplasie endométriale, dans lequel ledit extrait comprend du 3-butyl-4,5-dihydrophtalide et du 3-butyl-phthalide,

2. Extrait alcoolique selon la revendication 1, dans lequel le 3-butyl-4,5-dihydrophtalide est présent dans l'extrait en une quantité d'environ 95 % en poids et le 3-butyl-phtalide est présent dans l'extrait en une quantité d'environ 2 % en poids.

3. Extrait alcoolique du Rhizome Ligusticum Chuanxiong, **caractérisé en ce que** l'extrait est capable de déclencher une activité progestogénique dans un dosage des récepteurs aux progestatifs, pour une utilisation dans le traitement de l'accident vasculaire cérébral ou des lésions cérébrales chez un être humain ou un animal, dans lequel ledit extrait comprend du 3-butyl-4,5-dihydrophtalide et du 3-butyl-phtalide.

4. Extrait alcoolique selon la revendication 3, dans lequel le 3-butyl-4,5-dihydrophtalide est présent dans l'extrait en une quantité d'environ 95 % en poids et le 3-butyl-phtalide est présent dans l'extrait en une quantité d'environ 2 % en poids.
